# EUROPEAN PATENT APPLICATION

(11) **EP 4 095 283 A1**
(43) Date of publication of application: **30.11.2022**
(21) Application number: 21175835.4
(22) Date of filing: 25.05.2021
(51) Int. Cl.: C23C 16/04, A01N 25/10, A01N 25/34, A61L 31/10, A61P 31/12, B05D 1/00, C23C 16/452, C23C 16/455, C23C 16/513, A41D 13/11

(54) **METHOD AND SYSTEM FOR COATING FILTER MEDIA**

(71) Applicant: Molecular Plasma Group SA, 3895 Foetz (LU)
(72) Inventor: HEYBERGER, Régis, 57100 Thionville (FR); VANSANT, Jan, 3000 Leuven (BE); BOREK-DONTEN, Joanna, 6700 Arlon (BE); LOPES, Maximilien, 5170 Bois-de-Villers (BE)
(74) Representative: IPLodge bv

(57) **Abstract**

The present invention concerns a method for plasma coating a compressible structure , comprising the steps of: compressing the compressible structure, thereby at least partially, and preferably essentially completely, removing air from the compressible structure; and coating the compressed structure according to the following steps: a) ionizing a plasma gas at a temperature of 150°C or lower, and at about atmospheric pressure, thereby creating a plasma; b) introducing a precursor into said plasma, thereby obtaining a precursor-comprising plasma; c) exposing the compressed structure to said precursor-comprising plasma, thereby forming a coating onto surfaces of the structure.

## Description

### Technical field

The present invention concerns a method and system for coating an air-permeable filter media, as well as the coated filter media resulting therefrom. Hereto, the present invention uses an atmospheric pressure plasma coating technique, wherein a coating precursor is inserted in a plasma jet thereby creating a coating precursor-comprising plasma flow, and a filter media is subjected to said coating precursor-comprising plasma flow. The present invention is particularly apt at coating thick filters which may preferably be open or comprise open cells.

### Background

The current outbreak of the Sars-Cov-2 virus and Covid-19 disease show that there is need for a process which helps in preventing biological pathogens, in particular viruses, but also other biological pathogens such as bacteria, from spreading. These pathogens may be transferred by direct contact or by airborne transmission.

Typically, airborne transmission of a biological pathogen may occur e.g. via small droplets of bodily fluids carrying the pathogen, which are transported through the air, i.e. via an aerosol. Airborne transmission may also occur by the pathogen being transported in a gas-like state.

One way of preventing pathogens from spreading through air is by filtering the air. Hereby, the air can be made to flow through a filtering system comprising a filter made from an air-permeable material. The way the air is made to flow through the filter depends on the application. For instance, the air can be made to circulate using an air conditioning system, whereby a filter is positioned within one or more tubes. In another example, the filter is positioned in a facial mask, such that when someone breaths in or out, the resulting air flow passes through the filter in the facial mask.

Air-permeable filters may be very effective in stopping aerosols, i.e. in filtering out aerosols from an air flow. However, stopping the aerosol droplets may not be enough to prevent the pathogens from spreading. In particular, a filter may become saturated with pathogens in such a way that an air flow, which may not contain any pathogen, picks up pathogens from the filter. This can be the case in e.g. filters for air conditioning systems or for face masks.

In orderto protect against pathogens which are primarily captured in the filter from becoming airborne again, one solution is to treat the filter material, i.e. filter media, with a biological pathogen transfer inhibiting substance. Such biological pathogen transfer inhibiting substance is capable of destroying, inactivating and/or immobilizing the biological pathogen. The present invention is particularly apt against the spreading of viruses, in particular the Sars-Cov-2 virus. Note, however, that the normal operation of the filter may not be disturbed. Hence, the treated filter media must remain air-permeable without substantially reducing or even blocking the air flow compared to the untreated filter media. This is particularly important for filter media in HVAC systems (heating, ventilation and air conditioning), since the aeration and ventilation performance of the HVAC system should not be negatively affected by the coating, since proper ventilation and aeration is key to limiting the spread of pathogen containing aerosols within a building environment.

Document EP0859547B1 discloses reagents and methods for modifying a fabric substrate in order to inactivate viruses, and particularly lipid-enveloped viruses, upon contact. Such substrates can be modified by photochemically immobilizing hydrophilic polymers containing both quaternary ammonium groups and hydrocarbon chains, resulting in a localized surfactancy capable of disrupting lipid-enveloped viruses upon contact with the substrate. Substrates of the invention can be fabricated into the form of articles for medical and related use. This document relates to wet deposition, wherein a substrate is exposed to a solution containing the photopolymers.

Document WO2008127416A2 discloses hydrophobic polymeric coatings which can be non-covalently applied to solid surfaces such as metals, plastics, glass, polymers, textiles, and other substrates such as fabrics, gauze, bandages, tissues, and other fibers, in the same manner as paint, for example, by brushing, spraying, or dipping, to make the surfaces virucidal and bactericidal. Also here, the coating is applied by a wet method.

The coating techniques described above make use of 'wet coating', whereby a surface is subjected to a liquid solution containing the coating material. Such wet coating techniques generally suffer from a number of drawbacks such as:
- a long drying time which leads to a high energy consumption;
- large amount of waste resulting in a large impact on the environment;
- homogeneity and conformality of the coating is not always as desired,
- the thickness of the coating is not always under control, and may locally be much higher than desired,
- the coating may not penetrate a porous substrate deep enough;
- when using aqueous solutions, a large consumption of water.

Furthermore, it has been noticed that when a wet coating technique is used, post-rinsing is necessary and shows that a lot of the coating material still is rinsed away, i.e. much of the coating material is not well-adhered to the substrate. In the case a biocidal coating is envisioned, this may lead to unwanted health risks for users.

Also, wet coatings tend to be thick, i.e. of the order of 1 micrometer or more, which can result in a substantial reduction of air permeability.

A coating technique which has gained momentum in the last few decades is plasma coating. Hereby a precursor which is to form the coating on the surface of a substrate is brought at least partially in a plasma state, and the surface of the substrate is subjected to the plasmized precursor. As a result, the precursor can form strong bonds with the substrate and/or form cross-links between substance molecules, thereby resulting in a coating which may be thin, yet very durable, homogeneous and conformal. If the precursor is a polymerizable monomer, polymerisation may occur directly onto the surface of the substrate.

Plasma coating techniques may be divided into vacuum techniques, which have an operating pressure that is significantly lower than atmospheric pressure, and into atmospheric techniques which operate at or near atmospheric pressure, for instance 700 mbar and 1600 mbar, but preferably very close to atmospheric pressure, e.g. between 950 mbar and 1050mbar.

Plasma techniques to deposit a thin layer on a surface have been applied previously.

Document WO2019243631A1 discloses a method for plasma coating an object comprising an object profile, comprising the steps of: a) manufacturing a replaceable shield comprising a jet inlet, a nozzle outlet and a sidewall extending from the jet inlet to the nozzle outlet, wherein the nozzle outlet comprises an edge essentially congruent to at least part of the object profile; b) detachably attaching the replaceable shield to a jet outlet of a plasma jet generator; c) placing the object at the nozzle outlet such that the object profile fits closely to the nozzle outlet edge, thereby minimizing a gap between the nozzle outlet and the object; d) plasma coating the object with a low-temperature, oxygen-free plasma at an operating pressure which is higher than the atmospheric pressure, preferably by at most 10%, by providing a plasma jet in the shield via the plasma jet generator and injecting coating precursors in the plasma jet in the shield, thereby creating said operating pressure, thereby plasma coating the object in an oxygen-depleted plasma zone.

Document WO2019038378A1 discloses a method for depositing a coating on a substrate. A first precursor comprising fluoro-acrylate monomers, fluoro-alkyl acrylate monomers, fluoro-methacrylate monomers, fluoro-alkyl methacrylate monomers, fluoro-silane monomers, or a combination or derivates thereof is provided. A second precursor comprising linear siloxanes, silane monomers, cyclosiloxanes, cyclosilane monomers, or a combination or derivates thereof is provided. The first and second precursors are co-injected in a treatment region. An atmospheric or reduced pressure plasma discharge is created in said treatment region. The substrate coating comprises alternated multi-stacked nanostructures and is formed by copolymerization of the first and second precursors.

These documents describe a number of plasma techniques for depositing a layer onto a surface by producing a, preferably oxygen-poor or oxygen-free, plasma discharge and by introducing a coating precursor in the plasma discharge. The plasma discharge is then directed onto one or more surfaces of a substrate to be treated, thereby forming a coating.

The methods disclosed in these documents makes use of low-temperature plasma, typically at a temperature between 0°C and 100°C, and preferably at room temperature. The plasma discharge pressure is slightly higher than ambient pressure in order to ensure a directed plasma discharge and to ensure that oxygen present in the surrounding air is evacuated from the substrate surface during the treatment.

The present invention also aims to provide a method and system capable of coating an open structure, such as is typical for a filter. In fact, the present invention hereby aims also more generally to provide a method and system for coating a large variety of compressible structures.

### Summary of the invention

The present invention aims to provide a method and system for coating an air-permeable filter media, whereby the air-permeability is not substantially reduced due to the coating. The coating hereby inhibits airborne transfer of biological pathogens by destroying, inactivating and/or immobilizing the pathogen. The biological pathogen transfer inhibiting properties of the coating can be obtained by using a plasma coating precursor having the property of destroying, inactivating and/or immobilizing the pathogen.

The present invention relates to an atmospheric pressure plasma technique, which presents a number of advantages over vacuum plasma techniques, such as that no time-consuming depressurizing step is required and that both batch processing and inline processing, whereby the one or more objects which are to be treated, are sequentially treated, are easily achievable. Hence, substrates can be treated at a high throughput rate, while still allowing for a very smooth, thin coating.

In a first aspect of the invention, the present invention concerns a method for plasma coating an air-permeable filter media, comprising the steps of:
a) ionizing a plasma gas at low temperature, e.g. a temperature of 150°C or lower, and at about atmospheric pressure, thereby creating a plasma discharge;
b) introducing a coating precursor into said plasma discharge, thereby obtaining a coating precursor-comprising plasma discharge;
c) exposing the filter media to said coating precursor-comprising plasma discharge or to an afterglow resulting therefrom, thereby forming a coating onto a surface of the filter media,
whereby the coated filter media is air-permeable.

Hereby, the precursor comprises a biological pathogen transfer inhibiting compound, which preferably is a biological pathogen inactivation compound, a biological pathogen immobilisation compound and/or a biological pathogen proliferation decreasing compound. Preferred embodiments of inactivation compounds are a biocidal compound, a virucidal compound and/or a bactericidal compound.

Preferably, hereby, the air-permeability of the filter media coated using the method of the present invention is substantially the same as the air-permeability of the filter media prior to coating (untreated filter media). This is mainly because of the capability of a plasma to penetrate deep within the filter media material, in particular if the filter media comprises an open structure and/or an open cell structure, whereby the coating is deposited on the surfaces of the filter media structure reached by the plasma, both external surfaces as inner surfaces, thereby closely following the texture of these surfaces. Consequently, the coating as deposited using the present method does not form a layer across the openings or pores of the filter, and thus does not substantially reduce the air permeability.

The air permeability on textiles, such as filter media, can be measured according to ISO 9237 or ASTM D737, wherein the rate of flow of air passing perpendicularly through a given area of fabric is measured at a given pressure difference across the textile test area over a given time period (unit: cm³/(s.cm²)). Preferably, the decrease in air-permeability of the coated filter media compared to the untreated filter media is 15% or less, preferably 10% or less, more preferably 5% or less, such as 3% or less, 2% or less or even 1% or less.

Preferably, the pressure drop of the filter media coated using the method of the present invention is substantially the same as the pressure drop of the filter media prior to coating (untreated filter media). The pressure drop expresses the pressure differential ("delta P") across the filter media, i.e. the difference in pressure of a gas flow prior to and after passing the filter media. The term "substantially the same" with respect to the air-permeability, is intended to mean that the delta P of the filter media after coating does not increase significantly with respect to the delta P of the filter media prior to coating. Preferably, the increase in pressure drop of the coated filter media compared to the untreated filter media is 15% or less, preferably 10% or less, more preferably 8% or less, even more preferably 6% or less, such as 5% or less, 4% or less, 3% or less, 2% or less or, most preferably 1% or less.

In embodiments of the invention, the plasma coating has a thickness between 5 and 600 nm, preferably between 5 and 500 nm, more preferably between 10 and 500 nm, even more preferably between 10 and 300 nm, yet more preferably between 10 and 200 nm, still more preferably between 10 and 100 nm, most preferably about 20 nm. The plasma coating thickness can be well-controlled by controlling the exposure time of the filter media to the coating precursor-comprising plasma discharge.

The present invention also concerns an air-permeable filter media comprising a biological pathogen transfer inhibiting coating obtained using the method of the present invention. Preferably, the filter media is disposable.

The present invention also concerns a filter system comprising an air-permeably filter media according to the present invention, whereby the filter media is preferably replaceable. Preferably the filter media is disposable.

The present invention is particularly apt in treating thick filter media. The filter media can comprise a thickness of at least 1mm, more preferably at least 2 mm, still more preferably at least 3 mm, even more preferably at least 4 mm. Advantageously, the thickness of the filter media is between 2 mm and 20 mm.

Preferably, the filter media comprises a base weight of at least 50 g/m², preferably at least 75 g/m², more preferably at least 100 g/m², even more preferably at least 120 g/m². The base weight of preferred filter media utilized in the present invention is between 100 g/m² and 250 g/m², preferably between 120 g/m² and 200 g/m². The base weight corresponds to the weight per area of the filter media.

Advantageously, the filter media is a multilayer filter media, comprising a plurality of layers stacked onto one another. The plurality of layers are advantageously attached to one another, e.g. by lamination or needle stitching. The plurality of layers of the filter media can have different thickness and/or different density. The filter media preferably comprises one or more waved layers, e.g. having alternating peaks and valleys, wherein advantageously the height between a peak and a consecutive valley is larger than the thickness of the substrate constituting the waved layer. The waved layers can be undulated, or comprise a plurality of pleats. In addition, or alternatively, the filter media can comprise one or more flat layers, e.g. used as support or backing layer for the waved layers. It will be convenient to note that the values of thickness and base weight indicated hereinabove apply to the multilayer filter media as a whole.

Advantageously, the plurality of layers of the multilayer filter media are exposed to the plasma discharge or the plasma afterglow simultaneously. It has surprisingly been observed that the plasma coating methods as described herein allow to apply a coating into the depth of a multilayer filter media, hence obviating the need to plasma coat the different layers separately. In this respect, it can be beneficial when an outer layer of the multilayer filter media has a lower density compared to an inner layer of the multilayer filter media, to allow for a deeper penetration of the plasma activated species into the filter media.

Advantageously, the filter media has a particle retention efficiency according to ISO 16890 - ePM10 of at least 50%, preferably at least 70%, more preferably at least 80%. Advantageously, the filter media has a particle retention efficiency according to ISO 16890 - ePM1 of at least 50%, preferably at least 60%, preferably at least 70%, preferably at least 80%.

Preferably, the filter media comprises or consists of a (synthetic) polymer material. Examples of synthetic polymers are polyolefins, such as polypropylene (PP) or polyethylene, polyesters, such as polyethylene terephthalate (PET) or polybutylene terephthalate (PBT), polystyrene (PS), polyether, or polyimide. The filter media preferably comprises or consists of synthetic fibers, preferably arranged in one or more nonwoven layers. The filter media layer comprising fibres can be a nonwoven material, such as a meltblown nonwoven or a spunbond nonwoven. Preferably the filter media comprises or consists of polypropylene, more preferably meltblown polypropylene (MBPP) and/or spunbond polypropylene (SBPP).

In a particularly preferred embodiment, the coating precursor comprises citric acid. Citric acid may come in the form of colourless crystals of preferably 99.5 purity. In such case and in other cases, it is preferred to dissolve the citric acid, preferably in an aqueous solution, preferably in a concentration in the aqueous solution of between 10 wt% and 90 wt%, more preferably between 20 wt% and 80 wt%, still more preferably between 30 wt% and 70 wt%, yet more preferably between 40 wt% and 60 wt%, such as around 50 wt%.

Advantages of using a coating precursor comprising citric acid to deposit a coating on a surface of a filter media using methods of the present invention are that the virucidal and bacteriocidal properties of the deposited coating have proven to be durable. Furthermore, citric acid is known to be a safe substance for being used on air filter media, and is non-toxic to human beings, even in close proximity thereof and in contact therewith.

In an embodiment of the above methods, the precursor is administered in the plasma as a gas, as a liquid or as a solid, preferably as a gas or as a liquid in the form of an aerosol, most preferably as a liquid in the form of an aerosol.

In a second aspect, the present invention concerns a method for plasma coating a compressible structure , comprising the steps of:
- compressing the compressible structure, thereby at least partially, and preferably essentially completely, removing air from the compressible structure;
- coating the compressed structure according to the following steps:
   i) ionizing a plasma gas at low temperature and at about atmospheric pressure, thereby creating a plasma;
   ii) introducing a precursor into said plasma, thereby obtaining a precursor-comprising plasma;
   iii) exposing the compressed structure to said precursor-comprising plasma, thereby forming a coating onto surfaces of the structure.

The inventors have found that the presence of air inside the structure to be coated, may limit the quality of the coating. Without wishing to be bound by theory, the inventors believe that this could be due to two main reasons: the presence of the air at least partially prevents the precursor comprising plasma from penetrating the structure, and the presence of the oxygen in the air may lead to a quality decrease of the coating. Thus, by compressing the structure prior to exposing the structure to the precursor comprising plasma, the air is pushed out of the structure and the plasma is capable of deeply penetrating the structure. Furthermore, the absence of oxygen allows a better coating to be formed on the surface of the structure.

In a preferred embodiment, the compressible structure is mechanically compressed, more preferably by a set of rollers. This is particularly useful if the compressible structure comprises an in essence flat shape, such as a mat, a layer or a set of layers on top of each other. Preferably the compressible structure comprises, and more preferably is made of, a woven or non woven material.

In a preferred embodiment, the compressed structure decompresses during exposure of the compressed structure to the precursor comprising plasma, i.e. during step c.

In a preferred embodiment, the compressed structure is exposed to said precursor-comprising plasma from multiple sides simultaneously, preferably from at least two sides simultaneously, more preferably from at least two opposite sides. In the particularly preferred case of an essentially flat structure having an upper area side and a lower area side opposing the upper area side, the structure is exposed to said precursor-comprising plasma from both the upper area side and the lower area side. This can be achieved by a plasma apparatus comprising multiple outlets for the plasma and/or the precursor-comprising plasma, the outlets being directed towards the sides of the structures which need to be exposed, or simply by a multitude of plasma apparatuses, each configured to expose a predefined side of the structure to the precursor-comprising plasma. In this embodiment, the throughput of the plasma coating process can be higher and/or penetration of the structure by the precursor-comprising plasma is better, leading to a better coating.

The precursor preferably comprises a biological pathogen transfer inhibiting compound, which preferably is a biological pathogen inactivation compound, a biological pathogen immobilisation compound and/or a biological pathogen proliferation decreasing compound. Preferred embodiments of inactivation compounds are a biocidal compound, a virucidal compound and/or a bactericidal compound.

In a preferred embodiment, the compressible structure is air-permeable. Hereby, the air-permeability is substantially the same as the air-permeability of the structure prior to coating using the method of the present invention. This is mainly because of the capability of a plasma to penetrate deep within the compressible structure, in particular if the compressible structure is an open structure and/or an open cell structure, whereby the coating is deposited on the surfaces of the structure, thereby closely following these surfaces. Consequently, the coating as deposited using the present method does not form a layer across the openings or pores of the structure, and thus does not substantially reduce the air permeability. This is particularly preferred if the structure is an air permeable filter. Hence, in a particularly preferred embodiment, the compressible structure is an air-permeable filter.

The present invention also concerns a compressible structure comprising a coating, preferably a biological pathogen transfer inhibiting coating obtained using the method of the present invention. Preferably, the structure is made up of a disposable material. Preferably the compressible structure is an air permeable filter.

The present invention also concerns a system comprising a compressible structure according to the present invention, whereby the structure is preferably replaceable. Preferably the structure is disposable. This is particularly preferred if the structure is a filter and the system is a filter system.

The present invention is particularly apt in treating thick compressible structures. Hence, in an embodiment, the structure comprises a thickness of at least 1mm, more preferably at least 2 mm, still more preferably at least 3 mm, even more preferably at least 4 mm, yet even more preferably at least 5 mm, such as 6 mm, 7 mm, 8 mm, 9 mm, 10 mm, 11 mm, 12 mm, 13 mm, 14 mm, 15 mm, 16 mm, 17 mm, 18 mm, 19 mm, 20 mm. In an embodiment of the present invention, the structure comprises a thickness of at least 3 cm, more preferably at least 4 cm, still more preferably at least 5 cm, such as 6 cm, 7 cm, 8 cm, 9 cm, 10cm or more. This is particularly preferred if the structure is a filter.

Preferably, the structure comprises a base weight of at least 1 g/m², still more preferably at least 5 g/m², yet more preferably at least 10 g/m², even more preferably at least 15 g/m², and/or preferably at most 100 g/m², more preferable at most 60 g/m², still more preferably at most 50 g/m², such as 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 g/m² or any value there between. The base weight corresponds to the weight per area of the structure. Typically the structure hereby comprises an essentially homogenous thickness. This is particularly preferred if the structure is a filter.

Preferably, the structure comprises a polymer material. More preferably, the structure is made of polymer material. Preferably the structure comprises polypropylene, more preferably meltblown polypropylene (MBPP) and/or spunbond polypropylene (SBPP).

In a particularly preferred embodiment, the precursor comprises citric acid. Citric acid may come in the form of colorless crystals of preferably 99.5 purity. In such case and in other cases, it is preferred to dissolve the citric acid, preferably in an aqueous solution, preferably in aqueous solution of between 10wt% and 90 wt%, more preferably between 20 wt% and 80 wt% , still more preferably between 30 wt% and 70 wt%, yet more preferably between 40 wt% and 60 wt%, such as around 50 wt%. Citric acid has virucidal and bacteriocidal properties which remain after being deposited as a coating using the method of the present invention. Furthermore, citric acid is safe for being used on air filters and other structures which are used in close proximity of and/or in contact with a person. Also, citric acid is easily available.

### Brief discussion of the figures

Figure 1: at t=t0 the plasma is on. A coating precursor R-X is added to the plasma discharge and the coating precursor-comprising plasma discharge is contacted with the substrate. Hereby the coating precursor R-X is radicalized, and the surface of the substrate is activated.
Figure 2: at t=t1 the plasma is on. Radical recombination reactions are taking place on the surface of the substrate, resulting in a covalent bond between substrate and the coating precursor.
Figure 3: at t=t2, the plasma is on. Film growth and coating thickness depend on treatment time and the treatment parameters such as the power that is applied and the temperature. Also cross-linking is taking place.
Figure 4: at t=t3 the plasma is off. After the plasma treatment, a plasma deposited coating remains which is (preferably covalently) grafted onto the substrate.
Figure 5 represents a schematic cross section of a multilayer filter media with a plurality (three) waved layers and a backing layer.
Figure 6 represents a schematic cross section of a multilayer filter media with a plurality (two) waved layers and a backing layer.
Figure 7 represents a diagram of a multilayer filter media positioned in a plasma treatment apparatus.
Figure 8 represents a pocket filter comprising a multilayer filter media.
Figure 9 represents images of the four layers of a four-layer waved filter media used in aspects of the present disclosure.
Figure 10A represents a schematic cross section of a first multilayer filter media used in examples 2 and 3, composed of a waved layer and a backing layer.
Figure 10B represents a schematic cross section of a second multilayer filter media used in examples 2 and 3, composed of a superposition of two filter media of Fig. 9A.

### Detailed description of the invention

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The recitation of numerical ranges by endpoints includes all numbers and fractions subsumed within that range, as well as the recited endpoints.

The expression "% by weight", "weight percent", "%wt" or "wt%", here and throughout the description unless otherwise defined, refers to the relative weight of the respective component based on the overall weight of the formulation. The expression "% by volume", "volume percent", "%vol" or "vol%", here and throughout the description unless otherwise defined, refers to the relative volume of the respective component based on the overall volume of the formulation.

Whereas the terms "one or more" or "at least one", such as one or more or at least one member(s) of a group of members, is clear per se, by means of further exemplification, the term encompasses inter alia a reference to any one of said members, or to any two or more of said members, such as, e.g., any ≥3, ≥4, ≥5, 25 ≥6 or ≥7 etc. of said members, and up to all said members.

As used herein, the term antimicrobial refers to decreasing proliferation of microbial material, in particular viral and/or bacterial material, that may be present on a surface.

As used herein, the terms antiviral and virucidal refer to the capacity to destroy or inactivate viruses upon contact.

As used herein, the term aerosol refers to a suspension of fine solid particles or liquid droplets, in air or in another gas.

The term "biological pathogen" refers to a biological organism or compound which can produce disease. A pathogen may also be referred to as an infectious agent, or simply a germ. Preferably, the biological pathogen is a virus, a bacterium, a protozoan, a prion, a viroid, or a fungus. In a particularly preferred embodiment, the biological pathogen is a virus. In a preferred embodiment, inactivation of the biological pathogen is preferred.

A viral envelope is the outermost layer of many types of viruses. It protects the genetic material in their life-cycle when traveling between host cells. Not all viruses have envelopes. In an embodiment of the present invention, the biological pathogen is a lipid enveloped virus or a non-lipid-enveloped virus, preferably a lipid-enveloped virus.

Envelopes are typically derived from portions of the host cell membranes (phospholipids and proteins), but include some viral glycoproteins. They may help viruses avoid the host immune system. Glycoproteins on the surface of the envelope serve to identify and bind to receptor sites on the host's membrane. The viral envelope then fuses with the host's membrane, allowing the capsid and viral genome to enter and infect the host.

Some enveloped viruses also have a capsid, another protein layer, between the envelope and the genome.

The cell from which a virus buds often dies or is weakened, and sheds more viral particles for an extended period. The lipid bilayer envelope of these viruses is relatively sensitive to desiccation, heat, and detergents, therefore these viruses are easier to sterilize than non-enveloped viruses, have limited survival outside host environments, and typically must transfer directly from host to host. Enveloped viruses possess great adaptability and can change in a short time in order to evade the immune system. Enveloped viruses can cause persistent infections.

Examples of enveloped viruses:
- DNA viruses: Herpesviruses, Poxviruses, Hepadnaviruses, Asfarviridae
- RNA viruses: Flavivirus, Alphavirus, Togavirus, Coronavirus, Hepatitis D, Orthomyxovirus, Paramyxovirus, Rhabdovirus[2], Bunyavirus, Filovirus
- Retroviruses

The term "virus" according to the present invention includes double-stranded or single-stranded RNA or DNA viruses, which infect cells of bacteria, plants and/or animals. These include viruses from the following families of viruses: Iridoviridae, African swine fever virus, Poxyiridae, Parvoviridae, Reoviridae, Birnaviridae, Picornaviridae, Togaviridae, Flaviviridae, Rhabdoviridae, Bunyaviridae, Herpesviridae, Adenoviridae, Papovaviridae, Hepadnaviridae, Coronaviridae, Calicivirus, Arenaviridae, Paramyxoviridae, Orthomyxoviridae, Filoviridae, Retroviridae, Baculoviridae, Polydnaviridae, Nudaurelia (3 virus group, Nodaviridae, Caulimovirus, Geminivirus, Tomato spotted wilt virus group, Luteovirus, Machlovirus, Necrovirus, Sobemovirus, Tombusvirus, Tymovirus, Bromovirus, Cucumovirus, Ilarvirus, Alfafa mosaic virus group, Comovirus, Dianthovirus, Nepovirus, Pea enation mosaic virus group, Tobamovirus, Tobravirus, Hordeivirus, Potexvirus, Potyvirus, Carlavirus, Closterovirus, Totiviridae, Partitiviridae, Myoviridae, Styloviridae, Podoviridae, Tectiviridae, Plasmaviridae, Corticoviridae, Microviridae, Inoviridae, Cystoviridae and Leviviridae. In a preferred embodiment, the biological pathogen is a pathogen which infects human cells.

It should be understood that a virus may include viruses or infectious agents, which do not fall into the above mentioned families, e.g., plant satellite viruses, prions, baculoviruses and bacteriophage respectively.

The term "bacteriophage" according to the present invention is indicative of bacteriophage, which infect specific strains of bacteria e.g. salmonella, Escherichia coli, staphylococcus or pseudomonus bacteriophage. In an embodiment, the biological pathogen is a bacteriophage.

With respect to the first aspect of the invention, plasma coating techniques have been known in the prior art. For instance the PlasmaSpot^{®} and Plasmaline^{®} systems developed by Molecular Plasma Group S.A. are capable of performing the method of the present invention, provided they are supplied with the coating precursor comprising a biological pathogen transfer inhibiting compound as specified in this document, and provided the method is performed on a filter media. The air-permeability of the filter media is hereby substantially unaffected.

According to a second aspect of the invention, plasma coating techniques have been known in the prior art. For instance the PlasmaSpot^{®} and Plasmaline^{®} systems developed by Molecular Plasma Group S.A. are capable of performing the method of the present invention, provided that a structure compressing system for compressing the compressible structure is positioned before and/or within a plasma zone of the coating system such as the PlasmaSpot^{®} and Plasmaline^{®} systems developed by Molecular Plasma Group S.A. Such structure compressing system may preferably comprise a set of rollers. Such rollers can then be made to compress the compressible structure prior to entry into the plasma zone where the structure is exposed to the precursor comprising plasma. Preferably, the plasma apparatus is supplied with the precursor comprising a biological pathogen transfer inhibiting compound as specified in this document, and preferably the coating is performed on an air permeable structure more preferably an air-permeable filter. The air-permeability of the filter is hereby substantially unaffected.

If the invention concerns an air filter media, e.g. for an HVAC system, this air filter media is preferably coated with a viral suppressing agent to block the passage of airborne viruses. At the same time, air permeability of the filter material remains essentially unaffected by the coating process. It is particularly apt in decreasing the risk of the Sars-Cov-2 virus spreading via air or aerosols. Furthermore, although the viral suppressing agent is detrimental to the Sars-Cov-2 virus, it is environmentally friendly, as is the coating process. Hence, no additional treatment steps are required when the coated air filter media is disposed of.

The present invention therefore, in a first aspect, concerns a method for plasma coating an air-permeable filter media, comprising the steps of:
a) ionizing a plasma gas at a temperature of 150°C or lower, and at about atmospheric pressure, thereby creating a plasma discharge;
b) introducing a coating precursor into said plasma discharge, thereby obtaining a coating precursor-comprising plasma discharge;
c) exposing the filter media to said coating precursor-comprising plasma discharge, or to the plasma afterglow resulting therefrom, thereby forming a coating onto a surface of the filter media.

The coated filter media is air-permeable.

Hereby, the coating precursor comprises a biological pathogen transfer inhibiting compound, which preferably is a biological pathogen inactivation compound, a biological pathogen immobilisation compound and/or a biological pathogen proliferation decreasing compound. Preferred embodiments of inactivation compounds are a biocidal compound, a virucidal compound and/or a bactericidal compound.

The present invention also concerns a filter media coated using methods of the present invention, as well as filtering systems comprising said filter media.

The present invention concerns, in a second aspect, a method for plasma coating a compressible structure, comprising the steps of:
- compressing the compressible structure, thereby at least partially, and preferably essentially completely, removing air from the compressible structure;
- coating the compressed structure according to the following steps:
   i) ionizing a plasma gas at low temperature and at about atmospheric pressure, thereby creating a plasma;
   ii) introducing a precursor into said plasma, thereby obtaining a precursor-comprising plasma;
   iii) exposing the compressed structure to said precursor-comprising plasma, thereby forming a coating onto surfaces of the structure.

Preferably, herein the precursor comprises a biological pathogen transfer inhibiting compound, which preferably is a biological pathogen inactivation compound, a biological pathogen immobilisation compound and/or a biological pathogen proliferation decreasing compound. Preferred embodiments of inactivation compounds are a biocidal compound, a virucidal compound and/or a bactericidal compound.

The filter media can be a single layer material. Alternatively, in one aspect of the present invention, the filter media comprises two or more layers. The two or more layers can have the same or a different composition, i.e. comprising the same polymer(s) or different polymers. Further, the layers can have the same or a different structure, for example a first layer can be a meltblown polypropylene and a second layer can be a spunbond polypropylene. Further, the layers can have the same or a different orientation, in particular when the layers comprise fibres having a preferred orientation. Further, the layers can have the same or a different thickness.

In a preferred embodiment of the method, the coating precursor comprises a biocide compound, more preferably any compound or any combination of compounds in Table 1, wherein the structure is shown for easy reference. The chemical nature of the coating precursor can range from classic monomers to saturated molecules, from organic to inorganic molecules, from low molecular weight (e.g. monomers, oligomers) to high molecular weight (e.g. polymers being dissolved or emulsified).

**Table 1**

| Biocide name | CAS number | Structure |
|---|---|---|
| .alpha.,.alpha.',.alpha."-trimethyl-1,3,5-triazine-1,3,5(2H,4H,6H)triethanol (HPT) | 25254-50-6 | 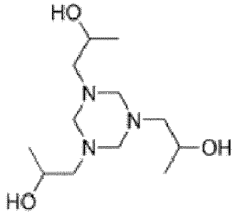 |
| Propan-1-ol | 71-23-8 | 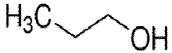 |
| Propan-2-ol | 67-63-0 | 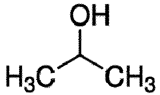 |
| 2-Phenoxyethanol | 122-99-6 | 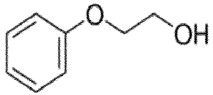 |
| Biphenyl-2-ol | 90-43-7 | 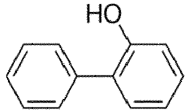 |
| Chlorocresol | 59-50-7 | 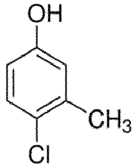 |
| Clorophene | 120-32-1 | 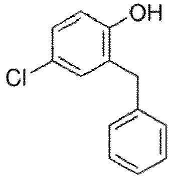 |
| 5-chloro-2-(4-chlorphenoxy)phenol (DCPP) | 3380-30-1 | 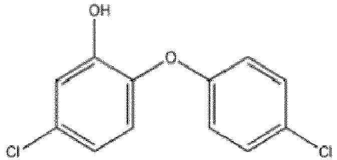 |
| D-gluconic acid, compound with N,N"-bis(4-chlorophenyl)-3,12-diimino2,4,11,13-tetraazatetradecanediamidine (2:1) (CHDG) | 242-354-0 | 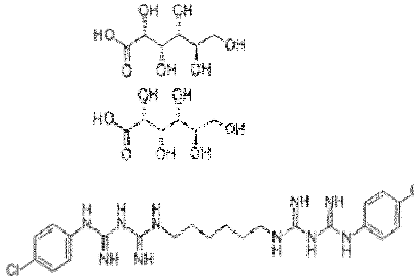 |
| 6-(phthalimido)peroxyhexanoic acid (PAP) | 128275-31-0 | 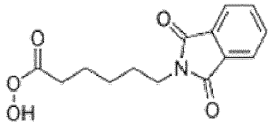 |
| Citric Acid | 77-92-9 | 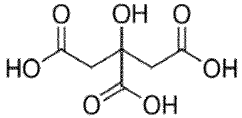 |
| Formic Acid | 64-18-6 | 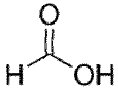 |
| Glycollic Acid | 79-14-1 | 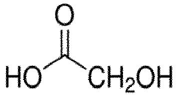 |
| L-(+)-lactic acid | 79-33-4 | 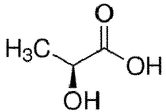 |
| Peracetic acid | 79-21-0 | 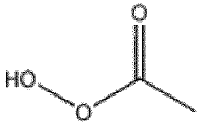 |
| Salicylic Acid | 69-72-7 | 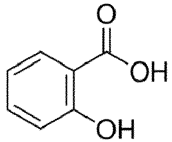 |
| Nonanoic Acid | 112-05-0 | 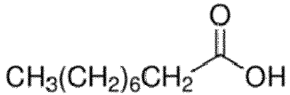 |
| Alkyl (C12-18) dimethylbenzyl ammonium chloride (ADBAC (C12-18)) | 68391-01-5 | 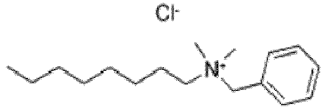 |
| Alkyl (C12-C14) ethylbenzylammonium chloride (ADEBAC (C12-C14)) | 85409-23-0 | 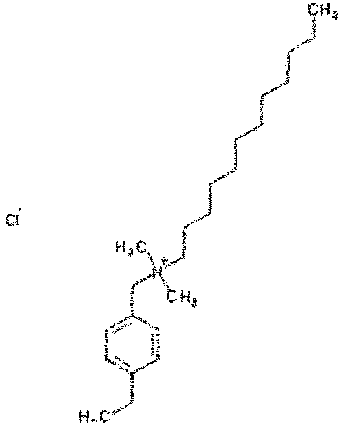 |
| Didecyldimethylammonium chloride (DDAC) | 7173-51-5 | 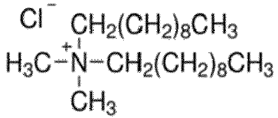 |
| Dimethyloctadecyl[3-(trimethoxysilyl)propyl] ammonium chloride (SiQAM) | 27668-52-6 | 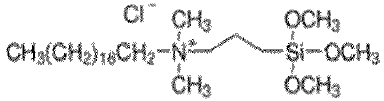 |
| Quaternary ammonium compounds, benzyl-C12-18-alkyldimethyl, salts with 1,2-benzisothiazol-3(2H)-one 1,1-dioxide (1:1) (ADBAS) | 68989-01-5 | 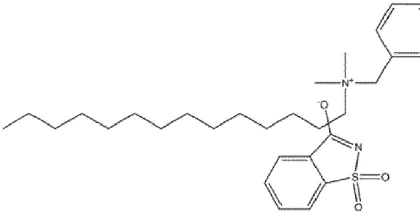 |
| N-(3-aminopropyl)-N-dodecylpropane-1,3-diamine (Diamine) | 2372-82-9 | 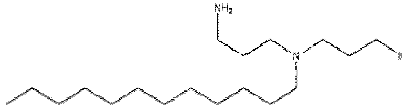 |
| Gluteraldehyde | 111-30-8 | 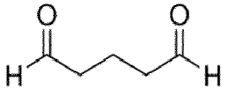 |
| Glyoxal | 107-22-2 | 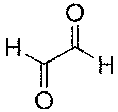 |
| Cinnamaldehyde / 3-phenyl-propen-2-al (Cinnamic aldehyde) | 104-55-2 | 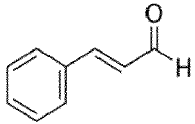 |
| Sodium dichloroisocyanurate dihydrate | 51580-86-0 | 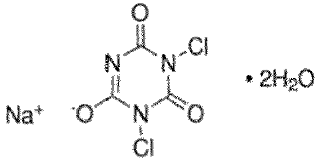 |
| Sodium N-chlorobenzenesulphonamide (Chloramine-B) | 127-52-6 | 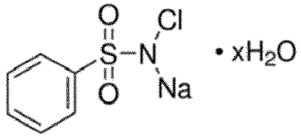 |
| Symclosene | 87-90-1 | 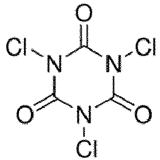 |
| Bromochloro-5,5-dimethylimidazolidine-2,4-dione (BCDMH/ Bromochlorodimethylhydantoin) | 32718-18-6 | 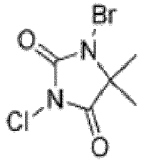 |
| Tosylchloramide sodium (Tosylchloramide sodium - Chloramin T) | 127-65-1 | 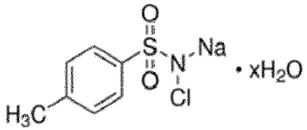 |
| Troclosene sodium | 2893-78-9 | 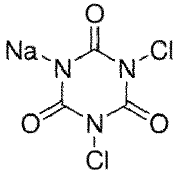 |
| Mixture of 5-chloro-2-methyl-2H-isothiazol-3-one (EINECS 247-500-7) and 2-methyl-2H-isothiazol-3-one (EINECS 220-239-6) (Mixture of CMIT/MIT) | 55965-84-9 | 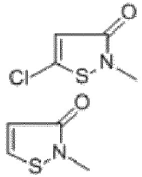 |
| Monolinuron | 1746-81-2 | 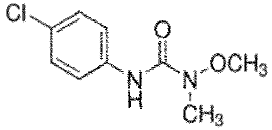 |
| Penta potassium bis(peroxymonosulphate) bis(sulphate) | 70693-62-8 | 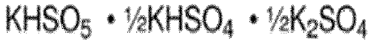 |
| Pyridine-2-thiol 1-oxide, sodium salt (Sodium pyrithione) | 3811-73-2 | 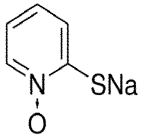 |
| Bronopol | 52-51-7 | 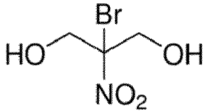 |
| Copper | 7440-50-8 | |
| 1,2-benzisothiazol-3(2H)-one (BIT) | 2634-33-5 | 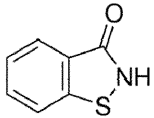 |
| 3,3'-methylenebis[5-methyloxazolidine] (Oxazolidin/MBO) | 66204-44-2 | 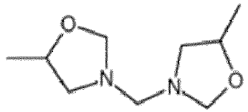 |
| Amines, N-C10-16-alkyltrimethylenedi-, reaction products with chloroacetic acid (Ampholyt 20) | 139734-65-9 | |
| 2-(Diethylamino)ethyl methacrylate (DIAMA) | 105-16-8 | 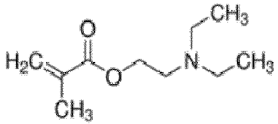 |
| 2-(tert-butylamino)ethyl methacrylate (BUTAMA) | 3775-90-4 | 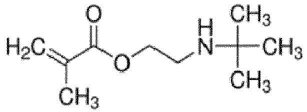 |
| 3-(Dimethylamino)-1-propylamine (DIMAP) | 109-55-7 | 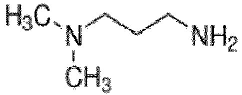 |
| N-[3-(N,N-dimethylamino)propyl]methacrylami de (DMAPMA) | 5205-93-6 | 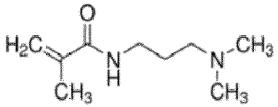 |
| PVP-I2 Poly(vinylpyrrolidone)-Iodine complex | 25655-41-8 | 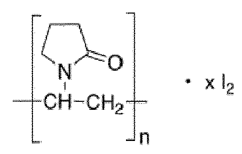 |
| Chitosan | 9012-76-4 | 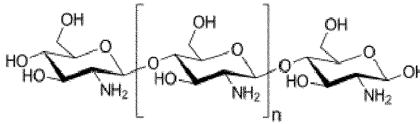 |
| Nisin | 1414-45-5 | |
| Natamycin | 7681-93-8 | |
| Chlorhexidine gluconate | 55-56-1 | |
| CuO nanoparticles | 1317-38-0 | |

Virucidal precursors are chemical substances such as individual compounds or compositions, attacking and inactivating, i.e. at least partially decreasing the infectivity of, the extracellular viral particles (virions). In many cases, virucidals (i) damage the virion protein capsid or supercapsidal membrane (when this shell is broken, there is no way for the virus to inject the material into the host cell), or (ii) penetrate into the virion and destroy the viral genome so that it can no longer replicate itself in the host. The viral particle integrity could also be affected.

In a preferred embodiment, the coating precursor comprises a virucidal compound. More preferably, the coating precursor comprises one or more of a hydrophobic precursor, a hydrophilic glycol-based precursor, an amino-based precursor, a sulphonate-based precursor, an ammonium-based precursor, a phosphonate-based precursor, a natural compound or any combination thereof, such as any one or any combination of the compounds in Table 2.

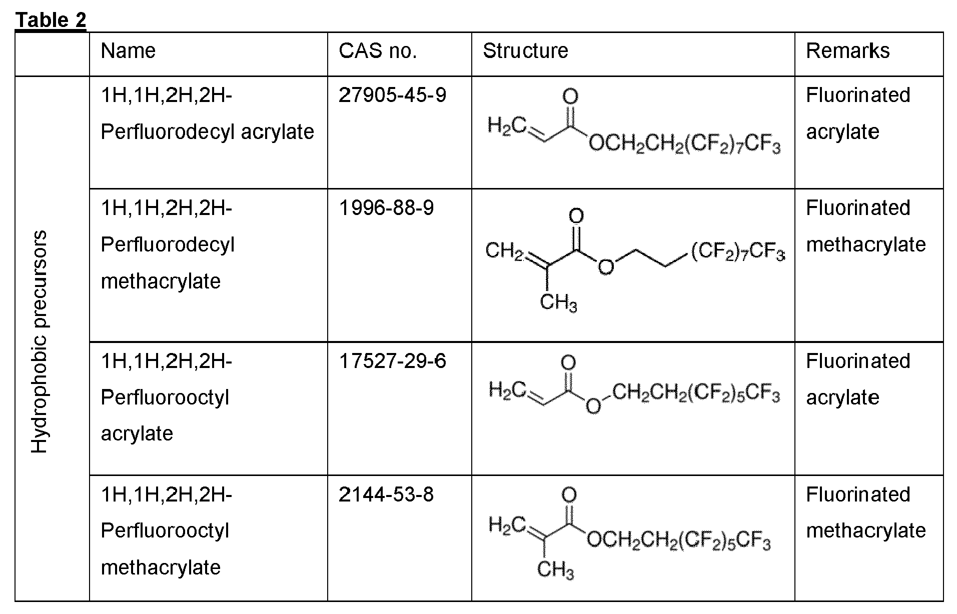

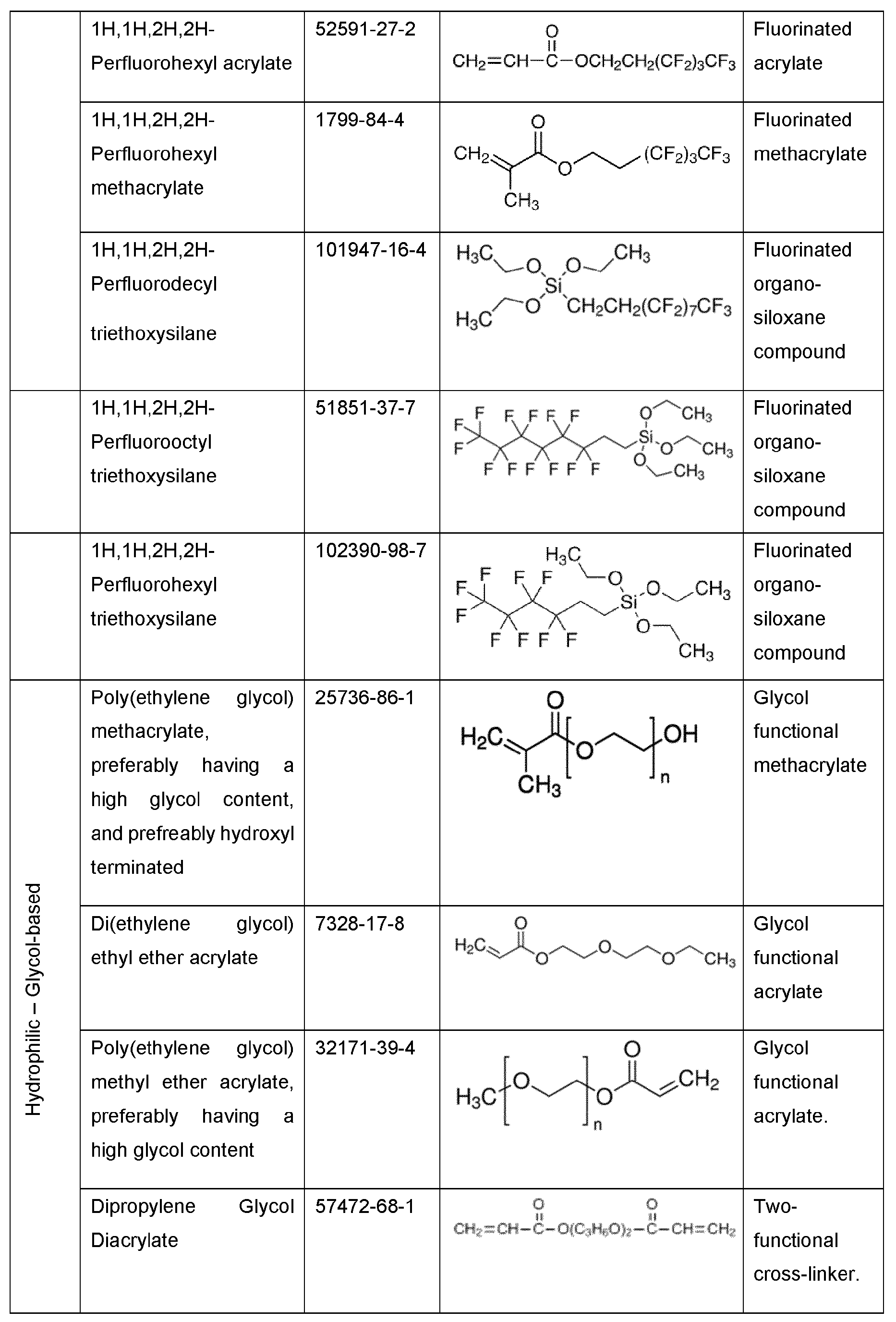

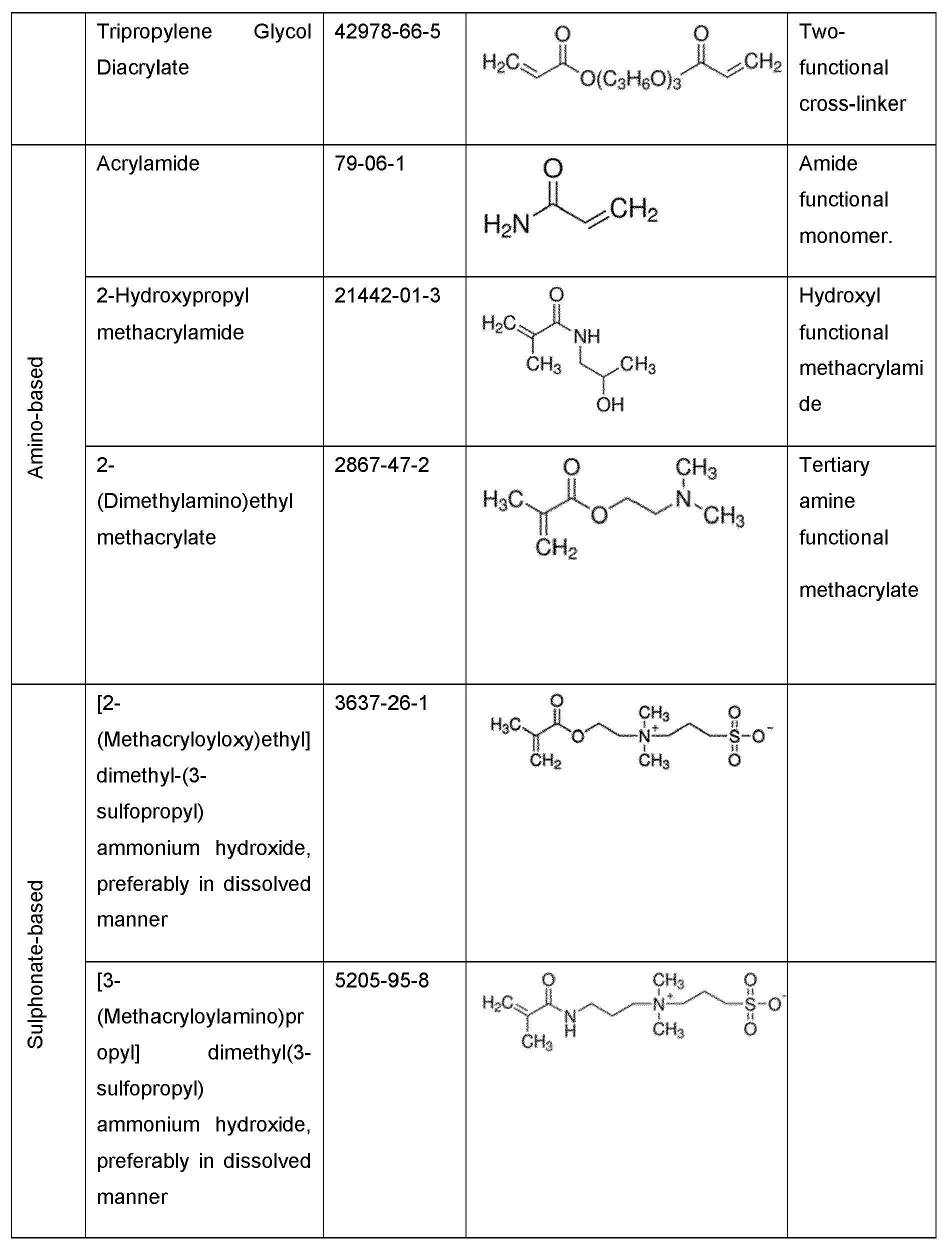

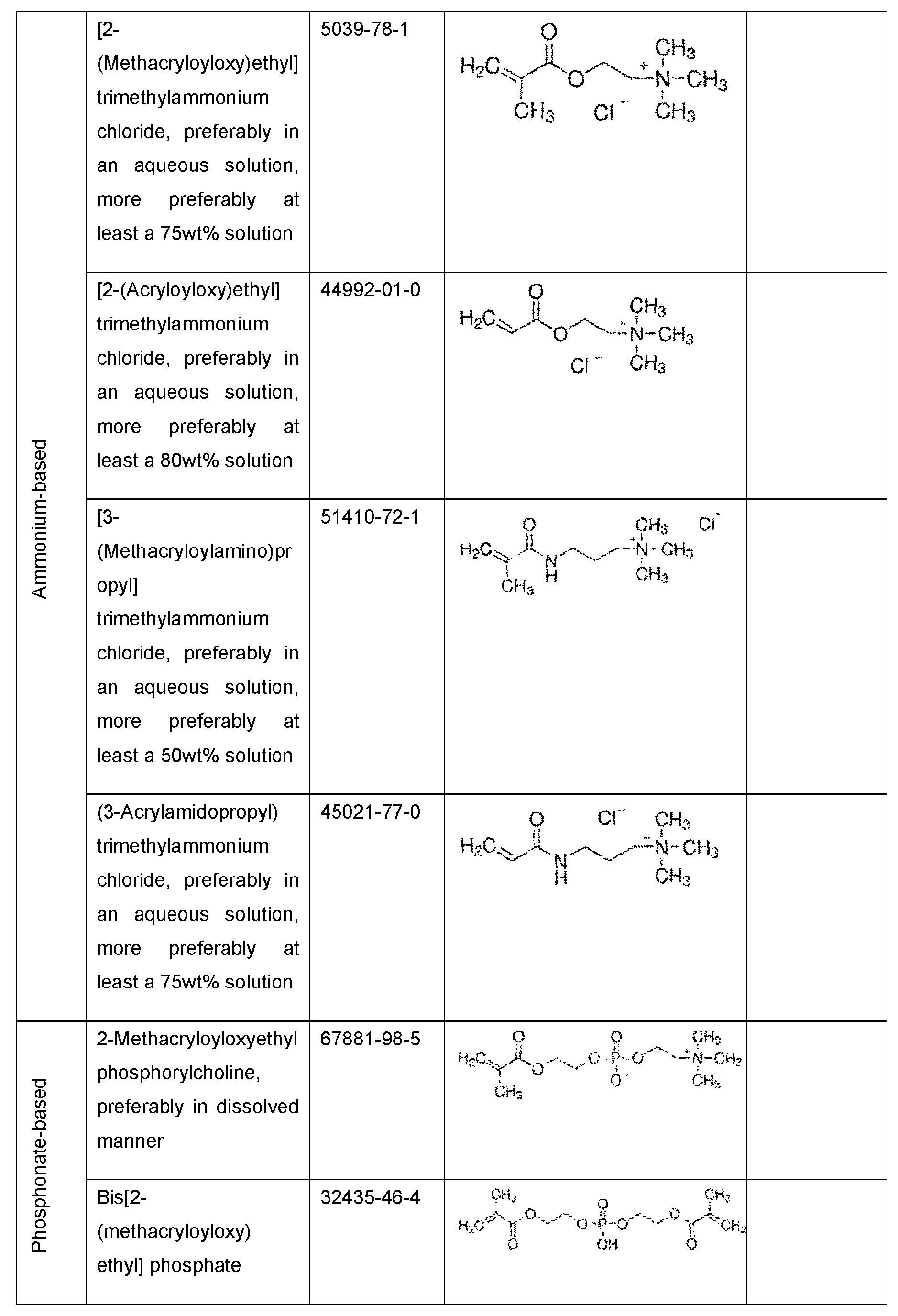

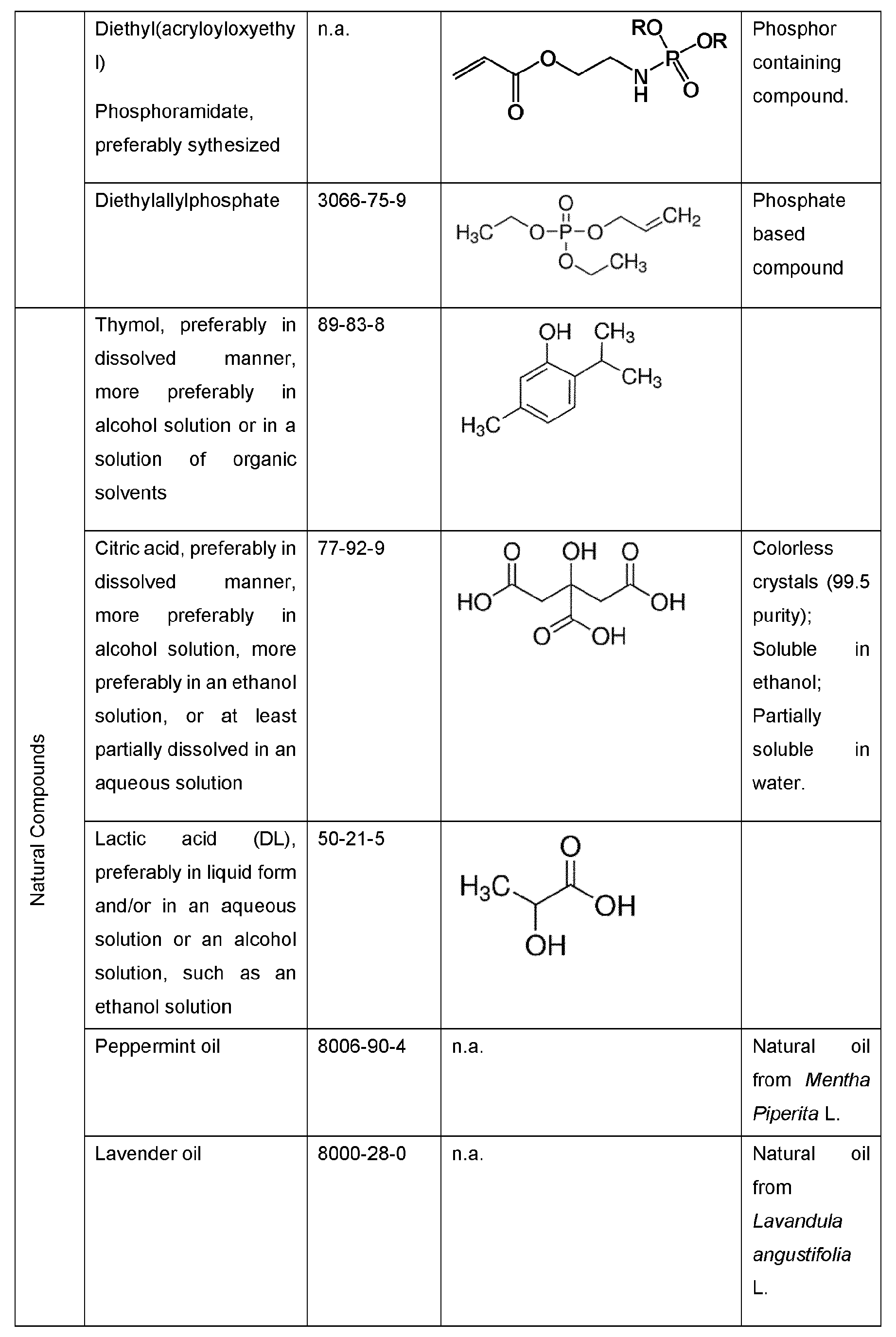

In a particularly preferred embodiment, the coating precursor comprises citric acid. Preferably, the precursor comprises at least 15% by weight of citric acid, based on the total weight of the precursor, such as at least 20% by weight, at least 30% by weight, at least 40% by weight, at least 50% by weight.

Citric acid may come in the form of colourless crystals of preferably 99.5% purity. Preferably, the citric acid is at least partially dissolved in an aqueous solution, such as water. Alternatively, the citric acid is dissolved in an alcohol-comprising solution, more preferably in an alcohol or in a combination of two or more alcohols. Examples of alcohols suitable to dissolve the citric acid are methanol, ethanol and propanol, such as isopropanol. Most preferably, the citric acid is dissolved in ethanol.

According to a preferred embodiment of citric acid as coating precursor, the solution comprising the at least partially dissolved citric acid, is introduced in the plasma discharge as an aerosol.

In an embodiment of the invention, one or more additional components are co-injected with the coating precursors into the plasma discharge. Specific examples of such additional components are inorganic particles, metallic particles or metallic oxide particles. With co-injection it is meant that the additional components can be mixed with the coating precursor prior to introduction into the plasma discharge, so that the additional particles and the coating precursor are introduced in the plasma discharge together. Alternatively, it is possible that the additional components are injected separately from but simultaneously with the coating precursor in the plasma discharge.

In an embodiment, the coating precursor further comprises an antibiotic and/or a peptide. In a preferred embodiment, the coating precursor further comprises Hydantoin (CAS no. 461-72-3), and/or one or more hydantoin derivatives.

The precursors preferably comprise any or any combination of the following compounds:
.alpha.,.alpha.',.alpha."-trimethyl-1,3,5-triazine-1,3,5(2H,4H,6H)triethanol (HPT),
Propan-1-ol, Propan-2-ol, 2-Phenoxyethanol, Biphenyl-2-ol, Chlorocresol, Clorophene, 5-chloro-2-(4-chlorphenoxy)phenol (DCPP),
D-gluconic acid, compound with N,N"-bis(4-chlorophenyl)-3,12-diimino2,4,11,13-tetraazatetradecanediamidine (2:1) (CHDG),
6-(phthalimido)peroxyhexanoic acid (PAP),
Citric Acid, Formic Acid, Glycollic Acid, L-(+)-lactic acid, Peracetic acid, Salicylic Acid, Nonanoic Acid,
Alkyl (C12-18) dimethylbenzyl ammonium chloride (ADBAC (C12-18)),
Alkyl (C12-C14) ethylbenzylammonium chloride (ADEBAC (C12-C14)), Didecyldimethylammonium chloride (DDAC),
Dimethyloctadecyl[3-(trimethoxysilyl)propyl] ammonium chloride,
Quaternary ammonium compounds, benzyl-C12-18-alkyldimethyl, salts with 1,2-benzisothiazol-3(2H)-one 1,1-dioxide (1:1) (ADBAS),
N-(3-aminopropyl)-N-dodecylpropane-1,3-diamine (Diamine),
Gluteraldehyde, Glyoxal, Cinnamaldehyde, 3-phenyl-propen-2-al (Cinnamic aldehyde), Sodium dichloroisocyanurate dihydrate, Sodium N-chlorobenzenesulphonamide (Chloramine-B), Symclosene,
Bromochloro-5,5-dimethylimidazolidine-2,4-dione (BCDMH/ Bromochlorodimethylhydantoin), Tosylchloramide sodium (Tosylchloramide sodium - Chloramin T), Troclosene sodium, Mixture of 5-chloro-2-methyl-2H-isothiazol-3-one (EINECS 247-500-7) and 2-methyl-2H-isothiazol-3-one (EINECS 220-239-6) (Mixture of CMIT/MIT),
Monolinuron,
Pentapotassium bis(peroxymonosulphate) bis(sulphate),
Pyridine-2-thiol 1-oxide, sodium salt (Sodium pyrithione),
Bronopol, Copper, 1,2-benzisothiazol-3(2H)-one (BIT),
3,3'-methylenebis[5-methyloxazolidine] (Oxazolidin/MBO),
Amines, N-C10-16-alkyltrimethylenedi-, reaction products with chloroacetic acid (Ampholyt 20) 2-(Diethylamino)ethyl methacrylate (DIAMA),
2-(tert-butylamino)ethyl methacrylate (BUTAMA),
3-(Dimethylamino)-1-propylamine (DIMAP),
N-[3-(N,N-dimethylamino)propyl]methacrylamide (DMAPMA),
PVP-I2 Poly(vinylpyrrolidone)-Iodine complex,
Chitosan,
Nisin,
Natamycin,
Chlorhexidine gluconate,
CuO nanoparticles.

In a particularly preferred embodiment, the precursors comprise citric acid. The above precursors, and particularly citric acid, are preferably used as a biological pathogen inactivation compound and are thus preferred in case a biocidal layer is desired.

Additionally or alternatively, the precursors may comprise a hydrophobic precursor, a hydrophilic glycol-based precursor, an amino-based precursor, a sulphonate-based precursor, an ammonium-based precursor, a phosphonate-based precursor, a natural compound or any combination thereof. More preferably, the precursors may comprise any or any combination of the following compounds: 1H,1H,2H,2H-Perfluorodecyl acrylate, 1H,1H,2H,2H-Perfluorooctyl acrylate (also referred to as 2-(Perfluorohexyl)ethyl acrylate), 1H,1H,2H,2H Perfluorodecyl triethoxysilane,
Poly(ethylene glycol) methacrylate, preferably having a high glycol content, and preferably hydroxyl terminated,
Di(ethylene glycol) ethyl ether acrylate,
Poly(ethylene glycol) methyl ether acrylate, preferably having a high glycol content, Dipropylene Glycol Diacrylate,
Tripropylene Glycol Diacrylate,
Acrylamide,
2-Hydroxypropyl methacrylamide,
2-(Dimethylamino)ethyl methacrylate,
[2-(Methacryloyloxy)ethyl] dimethyl-(3-sulfopropyl) ammonium hydroxide, preferably in dissolved manner,
[3-(Methacryloylamino)propyl] dimethyl(3-sulfopropyl) ammonium hydroxide, preferably in dissolved manner,
[2-(Methacryloyloxy)ethyl] trimethylammonium chloride, preferably in an aqueous solution, more preferably at least a 75wt% solution,
[2-(Acryloyloxy)ethyl] trimethylammonium chloride, preferably in an aqueous solution, more preferably at least a 80wt% solution,
[3-(Methacryloylamino)propyl] trimethylammonium chloride, preferably in an aqueous solution, more preferably at least a 50wt% solution,
(3-Acrylamidopropyl) trimethylammonium chloride, preferably in an aqueous solution, more preferably at least a 75wt% solution,
2-Methacryloyloxyethyl phosphorylcholine, preferably in dissolved manner, Bis[2-(methacryloyloxy) ethyl] phosphate,
Diethyl(acryloyloxyethyl),
Phosphoramidate, preferably synthesized,
Diethylallylphosphate,
Thymol, preferably in dissolved manner, more preferably in alcohol solution or in a solution of organic solvents,
Citric acid, preferably in dissolved manner, more preferably in alcohol solution, more preferably in an ethanol solution, or at least partially dissolved in an aqueous solution, Lactic acid (DL), preferably in liquid form and/or in an aqueous solution or an alcohol solution, such as an ethanol solution.
These precursors are preferably used as a biological pathogen inactivation compound wherein the biological pathogen is a virus, and thus in case a virucidal layer is desired.

In a preferred embodiment, the coating precursor comprises a halogen-containing compound, more preferably a halogen-leaching compound which is capable of leaching its halogen when present in the coating layer of the present invention. Preferably, the compound comprises chlorine, fluorine, bromine and/or iodine. Particularly preferred are halogenated Hydantoin or hydantoin-based compounds, such as, preferably, 1-Bromo-3-chloro-5,5-dimethylhydantoin (also called BCDMH or bromochlorodimethylhydantoin, CAS no. 16079-88-2). Without wishing to be bound by theory, it is expected that a biological pathogen coming into contact with a coating layer which leaches halogens, could be inactivated.

In a preferred embodiment, the precursors comprise succinimide or succinimide-based compounds. Succinimide (CAS no.: 123-56-8) has structural formula:

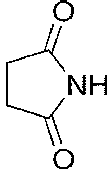

Succinimide and succinimide-based compounds are particularly preferred as biological pathogen immobilisation compounds.

In an embodiment, the precursors comprise any of the following:
- an organo-siloxane with a pathogen functional group;
- a polymerizable compound with a functional group, whereby preferably said polymerizable compound is an acrylate, a methacrylate or a vinyl, and/or
- a saturated compound with a functional group,
wherein the functional group is a biological pathogen transfer inhibiting functional group, which may be a fluorinated functional group, a glycol-based functional group, and amine group, a sulphonate-based functional group, an ammonium-based functional group or a phosphonate-based functional group. Preferably hereby, the amine group may be a primary amine group, a secondary amine group or a tertiary amine group. In a preferred embodiment, the precursors comprise FPDA and/or BUTAMA.

In an embodiment, the precursors comprise hydroxyl (alcohols), carboxyl (acids), aldehyde, amine, glycol, fluorocarbon, siloxane, quaternary ammonium, sulphonate, ammonium, phosphonate, halogens, natural oils, metals (metallic nanoparticles), metal oxides, inorganic particles, salts, enzyme, surfactant, peptide, lipopeptide, chitosan, antibiotics or any combination thereof.

In an embodiment of the present invention, the biological pathogen transfer inhibiting compound is a biological pathogen proliferation decreasing compound, preferably a microbial proliferation decreasing compound. The method of the present invention can then be used to decrease proliferation of microbial material adherent to the surface. Preferably hereby, the precursor comprises amine, siloxane, sulphonate, ammonium, phosphonate, quaternary ammonium, metal nanoparticles, enzyme, surfactant, peptide, lipopeptide, or any combination thereof.

In an embodiment of the present invention, the biological pathogen transfer inhibiting compound is a biological pathogen immobilisation compound, preferably a microbial collecting compound. The method of the present invention can then be used for collecting microbial material on the surface. Preferably hereby, the precursor comprises chitosan.

The present applicants have found that the abovementioned coating precursors significantly alter the surface properties of the filter media with respect to biological organisms and compounds, without significantly decreasing the air permeability and without significantly increasing the pressure drop. More particularly, the coating precursors are compounds which allow at least partial inhibition of transference of a biological pathogen which come into contact with a surface of the filter media provided with a layer deposited thereon using the method of the present invention. As indicated above, the at least partial inhibition of transference of the biological pathogen can be obtained in various ways, depending on the compound used as a precursor. At least three possible effects can be used to inhibit transfer of a biological pathogen.

Note that in the context of the present document, the term "inhibiting" includes a partial inhibition, in the sense that transfer of the biological pathogen is incomplete or slowed down. Preferably a complete inhibition of transfer is obtained.

The present invention is extremely apt at ensuring that the biological pathogen transfer inhibiting functionality of the precursor is maintained in the process of depositing the coating layer, and thus in the coating deposited on the surface(s) of the filter media. This is realised because the plasma gas is applied at standard conditions of temperature and pressure, e.g. at or around room temperature and at or around atmospheric pressure, and because the precursor is introduced into the plasma discharge, rather than being plasmized directly, i.e. the precursor is mainly indirectly excited through collisions with plasma species. This type of excitation decreases the risk of fragmentation of the precursor or any other possible cause of functionality loss.

It is important to realize that the filter media onto which the coating is deposited can have any type of shape and size, and can be difficult to treat, due to their inert nature or their extreme fragility, e.g. natural materials, biodegradable or water soluble materials. However, the method of the present invention is remarkably gentle and can be used on a multitude of materials.

In an embodiment of the above methods, the precursor is administered in the plasma discharge as a gas, a liquid or a solid, preferably as a gas or as a liquid in the form of an aerosol, most preferably as a liquid in the form of an aerosol.

Preferably a low-energy plasma is used in the present invention. A low-energy plasma is defined herein as a plasma of which the power density is high enough to activate the precursor and/or the substrate, i.e. the filter media, allowing a chemical reaction to take place, but low enough to prevent destruction of the precursor and the substrate. The power density is preferably in the range of 0.2 W/I (W per litre volume of the chamber where the reaction takes place) to 8W/I, more preferably between 0.5 W/I and 7 W/I, still more preferably between 0.8 W/I and 6 W/I, yet more preferably between 1 W/I and 5 W/I, even more preferably between 1.5 W/I and 4 W/I, still even more preferably between 2 W/I and 3 W/I, such as 2 W/I, 2.1 W/I, 2.2 W/I, 2.3 W/I, 2.4 W/I, 2.5 W/I, 2.6 W/I, 2.7 W/I, 2.8 W/I, 2.9 W/I, 3 W/I or any value therein between, most preferably in the range of 2.4W/I to 2.6 W/I.

Preferably a cold plasma is used in the present invention. A cold plasma is defined herein as a plasma of which the temperature is sufficiently low to avoid any damage to the precursor (e.g. fragmentation) and/or the filter media (e.g. melting) that are exposed to said cold plasma. Advantageously, the temperature of the plasma discharge is 150°C or lower, more preferably 130 °C or lower, still more preferably 100 °C or lower, yet more preferably 70°C or lower, even more preferably 60°C or lower, yet more preferably 55°C or lower, still even more preferably 50°C or lower, even yet more preferably 45°C or lower. The temperature of the plasma discharge may preferably be as low as room temperature, i.e., the temperature surrounding the plasma discharge. Depending on the location where the coating process is carried out, room temperature may be in the range of 10 to 40 °C, preferably 15-30 °C, such as 20-25 °C. The temperature of the plasma discharge will generally not be lower than room temperature.

When depositing temperature sensitive coatings it is important to keep the temperature of the plasma steady at the optimal value. Depending on the type of precursor or precursor mixture and/or the pressure, the optimal temperature may be selected. Hence, in an embodiment the temperature of the plasma is selected taking into account the type of precursor, the precursor mixture and/or the plasma pressure.

The plasma of the present invention is preferably an atmospheric pressure plasma which has a pressure around ambient pressure. Such plasma is created and discharged at a pressure of between 500 and 1300 hPa, preferably between 600 and 1250 hPa, even more preferably between 700 hPa and 1200 hPa, still more preferably between 800 hPa and 1150 hPa, yet more preferably between 900 hPa and 1100 hPa, most preferably about ambient pressure, which can typically be about 1013 hPa. Note that low-energy, cold plasma can also be applied under reduced pressure of lower than 400 hPa down to vacuum, or increased pressure of more than 1600 hPa, both types requiring a pressure vessel to maintain such low or high pressures. However, the use of a plasma with pressures in the currently preferred ranges around the ambient pressure reduces any costs and difficulties relating to maintaining pressure differences and pressure gradients.

In a preferred embodiment, the plasma is a dielectric barrier discharge plasma under atmospheric pressure.

In a preferred embodiment, the plasma gas is ionized by means of electrodes, whereby more preferably said plasma gas is ionized by said electrodes at a power of at most 10 Watt per cm² of the electrode surface, more preferably at most 9 W/cm², still more preferably at most 8 W/cm², even more preferably at most 7.5 W/cm². In many embodiments of the present invention, the power applied by the electrodes is minimally 1 W/cm², preferably minimally 2 W/cm², still more preferably minimally 2.5 W/cm². The power is most preferably between 2.5 and 7.5 W/cm².

In a preferred embodiment, the plasma gas comprises inert gas for at least 99 % by volume. The use of an inert gas as plasma gas essentially ensures that no side-reactions take place with the plasma gas and the equipment, between molecules of the plasma gas themselves, even not if temperature is increased. In fact, the lack of side-reactions also seems to allow to keep the processing temperature low, e.g. less than 50°C and preferably around room temperature. The low temperature of the plasma allows treatment of substrates made from a wide range of materials. Furthermore, this allows a better control over the formed coating and the adhesion properties thereof. Without wishing to be bound by theory, the inventors believe that the lack of reactive gas in the plasma gas ensures that none to very few chemical reactions with the plasma gas take place at the surface of the substrate, hence the better control over the coating properties. Also, if the plasma gas is nitrogen (N₂) or is mainly comprised of N₂, the low power applied to the plasma in embodiments of the present invention, are seen to result in very little to none nitrogen incorporated in the resulting coating. This is in stark contrast with the use of e.g. O₂, NH₃ or CH₄ as a plasma gas, all of which are deemed reactive gasses, and all of which seem to leave more traces within the coating of the plasma gas, thereby leading to loss of control over the coating properties.

In a preferred embodiment, said plasma gas comprises inert gas for at least 99 % by volume, i.e. 1 % by volume (vol.%) or less of the plasma gas is a reactive gas. More preferably at least 99.5 vol%, still more preferably at least 99.8 vol%, still more preferably at least 99.9 vol%, even more preferably at least 99.95 vol%, yet more preferably at least 99.99 vol% of the plasma gas is an inert gas. This means that the plasma gas preferably comprises 1 vol.% or less O₂, more preferably at most 0.5 vol%, still more preferably at most 0.2 vol%, yet more preferably at most 0.1 vol%, still more preferably at most 0.05 vol%, even more preferably at most 0.01 vol% of O₂. In the atmospheric plasma process of the present invention, this can for instance be achieved by using an overpressure with respect to ambient pressure, e.g. the plasma gas is delivered at a pressure of at least 1013mbar, preferably at least 1020mbar, more preferably at least 1030mbar, even more preferably at least 1040mbar, still more preferably at least 1050 mbar. Such slight overpressures allow to create an oxygen-poor and even oxygen-free zone in the plasma afterglow.

In a preferred embodiment, the precursor is added in a plasma gas afterglow. Hereby plasma gas flows over and between a plasma-inducing system, e.g. a set of electrodes. Downstream of the plasma-inducing system, a plasma gas afterglow is present, which comprises a large number of excited plasma species, such as ionized plasma gas molecules which did not have the time to deionize or plasma gas molecules in an excited state. The precursor is preferably introduced in said plasma gas afterglow. As a result, the precursor does not need to be introduced in between e.g. electrodes which are used to ionize the plasma gas, and thus the electrodes may be kept clean for a long duration as the precursor cannot form a layer onto the electrodes.

The atmospheric pressure plasma coating processes of the present invention allows both batch processes and inline processes. Hence, in an embodiment, the structure, such as the filter media, moves during the step of exposure of the structure, such as the filter media, to the coating precursor-comprising plasma discharge. In another embodiment, the structure, such as the filter media, is static during the step of exposure of the structure, such as the filter media, to the coating precursor-comprising plasma discharge. In yet another embodiment, the structure, such as the filter media, moves and remains static during the step of exposure of the structure, such as the filter media, to the coating precursor-comprising plasma discharge according to a predetermined trajectory. This allows to provide e.g. a thicker coating on some portions of the structure, such as the filter media, and thinner coating on other portions of said structure, such as said filter media.

In an embodiment of the invention, the plasma gas flow is between 1 and 1500 standard liter per minute ("slm"), more preferably between 50 and 1500 slm. 1 "slm" is a liter of the gas at atmospheric pressure and at room temperature. More preferably the plasma gas flow is between 80 slm and 1000 slm, such as between 100 slm and 750 slm, between 125 slm and 600 slm, or between 150 slm and 500 slm.

Preferably the plasma gas comprising the precursor is jettisoned from an outlet of a plasma jet nozzle. In a preferred embodiment, the plasma gas flow is determined taking into account a distance between the surface of the substrate and the outlet of a plasma jet nozzle. The larger such distance, the more plasma gas flow is required to ensure that the surface of the filter media is subjected to a plasma without reactive gasses other than the used precursor. In particular, one can ensure that the plasma is essentially free of oxygen coming from e.g. the surrounding air.

In an embodiment of the present invention, the structure, such as the filter, undergoes a plasma pre-treatment step prior to being subjected to the plasma comprising the precursor. Hereby, the plasma pre-treatment preferably activates the surface of the structure, such as the filter, i.e. it removes microscopic organic contamination and/or generates surface radicals, and may also preferably at least partially oxidize the surface, leading to an increased surface energy in most cases.

In preferred embodiments, the pre-treatment is performed:
- in an oxygen-rich plasma environment, more preferably using air or CO₂ or other oxygen containing species,
- at higher power compared to the power used during the plasma gas ionization step a), and/or
- without the addition of chemical precursors.

In embodiments of the present invention, the coated structure, such as the filter media, undergoes an atmospheric plasma post-treatment step. Preferably during this post-treatment step, the molecular weight of the coating is increased and/or the thermal stability of the coating is improved, for example by further cross-linking of the deposited coating.

In preferred embodiments, the plasma post-treatment is performed:
- in absence of oxygen, using inert plasma gases, such as N₂, Ar, of He (or mixtures thereof);
- at lower plasma power than the power used during the plasma gas ionization step a), and/or
- without the addition of chemical precursors.

The plasma chemically activates the coating precursor and/or the surface(s) of the filter media. This activation of the precursor and/or the surface may occur by double atomic bonds opening, radical removal and/or ion formation. This allows and/or improves the reactions required to form the coating layer. These reactions may involve:
- reactions between precursor molecules, such as polymerization reactions and cross-linking reactions, and/or
- reactions between precursor molecules and the surface, such as covalent bonding reactions. Preferably, the coating is covalently grafted to the surface.

In embodiments of the invention, the plasma coating has a thickness between 5 and 600 nm, preferably between 5 and 500 nm, more preferably between 10 and 500 nm, even more preferably between 10 and 300 nm, yet more preferably between 10 and 200 nm, still more preferably between 10 and 80 nm, such as 10 nm, 20 nm, 30 nm, 40 nm, 50 nm, 60 nm, 70 nm, 80 nm or any value therein between, most preferably about 20 nm. The plasma coating thickness can be well-controlled by controlling the exposure time of the surface to the coating precursor-comprising plasma discharge and/or the process parameters, such as temperature, pressure and power applied.

Preferably, the air-permeability of the filter media coated using the method of the present invention is substantially the same as the air-permeability of the filter media prior to coating (untreated filter media). The air permeability can be measured according to ISO 9237 or ASTM D737, wherein the rate of flow of air passing perpendicularly through a given area of fabric is measured at a given pressure difference across the textile test area over a given time period (unit: cm³/(s.cm²)). Preferably, the decrease in air-permeability of the coated filter media compared to the untreated filter media is 20% or less, preferably 15% or less, more preferably 10% or less, such as 9% or less, 8% or less, 7% or less, 6% or less, or 5% or less, 4% or less, 3% or less, 2% or less, or even 1% or less.

Preferably, the flow resistance of the filter media coated using the method of the present invention is substantially the same as the flow resistance of the filter media prior to coating (untreated filter media). The flow resistance is expressed as a pressure differential ("delta P") across the filter media, i.e. the difference in pressure across the filter media of a gas, such as air, that is made to flow at a controlled flow rate and/or flow speed. Flow resistance of a filter media can be tested with an automated filter tester type 8130, TSI Incorporated. The term "substantially the same" is intended to mean that the delta P of the filter media after coating does not increase significantly with respect to the delta P of the filter media prior to coating. Preferably, the increase in flow resistance of the coated filter media compared to the untreated filter media is 20% or less, preferably 15% or less, more preferably 10% or less, such as 9% or less, 8% or less, 7% or less, 6% or less, such as 5% or less, 4% or less, 3% or less, 2% or less, or 1% or less. Preferably, the flow resistance of the plasma coated filter media according to aspects of the present invention is 120 Pa or less, advantageously 100 Pa or less, advantageously 90 Pa or less, advantageously 80 Pa or less, advantageously 70 Pa or less.

The decrease in air permeability and the increase in flow resistance of the coated filter media compared to the untreated filter media is related to both the coating thickness and the structure of the filter media. A thicker coating can reduce the air permeability and increase the pressure drop to a higher degree than a thinner coating, especially when the filter media has rather small pores.

The applicant has found that with the methods of the invention a coated filter media is obtained with a limited decrease in air permeability and limited increase in flow resistance compared to the untreated filter media, in combination with excellent virucidal and anti-bacterial properties. This combination of properties is even obtained for filter media having thicknesses of 2 mm or more. The coating precursors used in the present invention have proven to provide excellent virucidal and anti-bacterial properties to a substrate by means of thin coatings having a thickness of 600 nm or less. Further, by using the process parameters of the methods of the invention, the applicant has discovered that the thin virucidal and anti-bacterial coatings are deposited into the depth of the filter media while allowing to maintain the openness of the filter media, and hence limit the reduction of the air permeability and the increase in the pressure drop.

In a preferred embodiment, the processing temperature is at most 50°C, more preferably at most 40°C, still more preferably at most 30°C and most preferably around room temperature.

In a preferred embodiment, the processing temperature is controlled, more preferably by heating or cooling one or more of the electrodes used for ionizing the plasma gas. The temperature of the electrodes can be regulated by passing air or a liquid, such as water or oil, through the electrode(s). Preferably the temperature of the electrodes and/or the filter media (or the substrate holder when the filter media is placed on a substrate holder in the plasma treatment apparatus) is measured in order to allow better control the temperature of the plasma gas. Typically this can be achieved by using a temperature control system, e.g. a PID controlling system, to check how a predetermined desired processing temperature relates to the measured temperature, which allows to steer the cooling/heating of the plasma discharge, e.g. by cooling/heating the electrode(s), the filter media, and, when used, the substrate holder. Optionally, one or more walls of the plasma discharge chamber of the apparatus can be temperature controlled as well. Preferably, the temperature control system ensures that the processing temperature lies between the electrode temperature and the filter media (substrate) temperature.

The plasma deposition process of the present invention is based on the simultaneous generation of surface radicals (i.e. activation of the difficult-to-treat substrate) and radicalized species in the plasma gas phase, leading to radical recombination reactions of the species to the substrate (i.e. grafting based on covalent bonding).

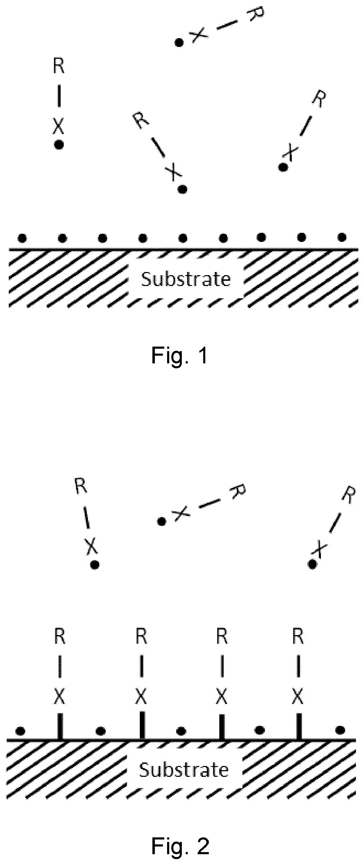

The scheme outlined in figures 1 to 4 indicates the different phases during the atmospheric pressure plasma deposition process:
**Figure 1****:** at t=t0 the plasma is on. A coating precursor R-X is added to the plasma gas and the coating precursor-comprising plasma discharge is contacted with the surface of the substrate (filter media). Hereby the precursor R-X is radicalized, and the surface is activated.
**Figure 2****:** at t=t1 the plasma is on. Radical recombination reactions are taking place on the surface, resulting in a covalent bond between the surface of the substrate and the coating precursor.

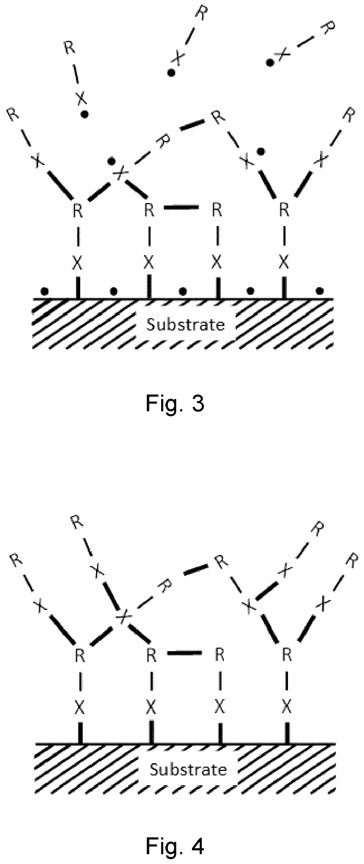

**Figure 3****:** at t=t2, the plasma is on. Film growth and coating thickness depend on treatment time and the treatment parameters such as the power that is applied and the temperature. Also cross-linking is taking place.
**Figure 4****:** at t=t3 the plasma is off. After the plasma treatment, a functional plasma deposited coating (film) remains which is grafted, preferably covalently grafted, onto the surface of the substrate.

In Step 1, the plasma is generated (can be based on direct or indirect plasma configurations, using an inert plasma gas such as N2, Argon, Helium, or any mixtures thereof), instantaneously generating radicalized species in the plasma gas phase. These species can be added to the plasma as a gas (or gas mixture), or a liquid (e.g. an aerosol, a spray, a liquid mixture, an emulsion, a dispersion, or polymer solution), preferably as a gas or as an aerosol. In the scheme outlined in figs. 1-4, the connotation "R-X" is used to denote the initial coating precursor, and "R-X•" to denote the radicalized form of the precursor, "R" being the targeted functionality, and "X" being a part of the molecule being able to be radicalized. For example, "X" can be reactive (such as C=C double bonds, C=O, epoxy, isocyanate, etc.), but can also be unreactive (i.e. saturated). In this specific case, the radical will be formed based on hydrogen abstraction or any other single bond scission.

In addition to the radicalized species in the gas phase, also surface radicals are formed on the surface of the substrate (filter media) when it is in contact with the plasma discharge. The generation of these surface radicals can be mainly based on hydrogen abstraction or breaking of covalent bonds located at the surface of the substrate.

In Step 2, radical recombination reactions are taking place between the radicalized species and surface radicals. This radical recombination reaction results in a permanent grafting of the precursor to the surface of the filter media by the formation of a covalent bond. It must be remarked that presence of reactive gasses, such as O₂, preferably is avoided during this phase.

In Step 3, film growth is taking place by the continuous incorporation of species by radical recombination. It must be remarked that the plasma process is 'non-specific', meaning that a specific precursor can be built in on the surface on any location, leading to a heterogeneous conformation of the plasma deposited film on a molecular level. Furthermore, the film growth can take place in a 'continuous' plasma or in a 'pulsed' plasma process. This pulsed plasma has a specific plasma off time during which no power is applied and wherein recombination reactions are favoured, similar to propagation in conventional polymer synthesis.

In the final phase of the plasma deposition process (Step 4), the plasma is switched off, or similarly the substrate has left the plasma afterglow zone, leading to a fully functional coating layer which is covalently linked to the surface of the substrate.

It has been observed that the biological pathogen transfer inhibition characteristics of the layer formed by using a precursor with a biological pathogen transfer inhibition compound can be reached under a large range of operational conditions. Note hereby that the function of inhibiting transfer of a biological pathogen is relatively crude, in the sense that it does not require an intricate catalytic. The main idea of the present invention is to reduce the transfer of the biological pathogen, not to induce intricate chemical reactions on the surface of the filter media substrate.

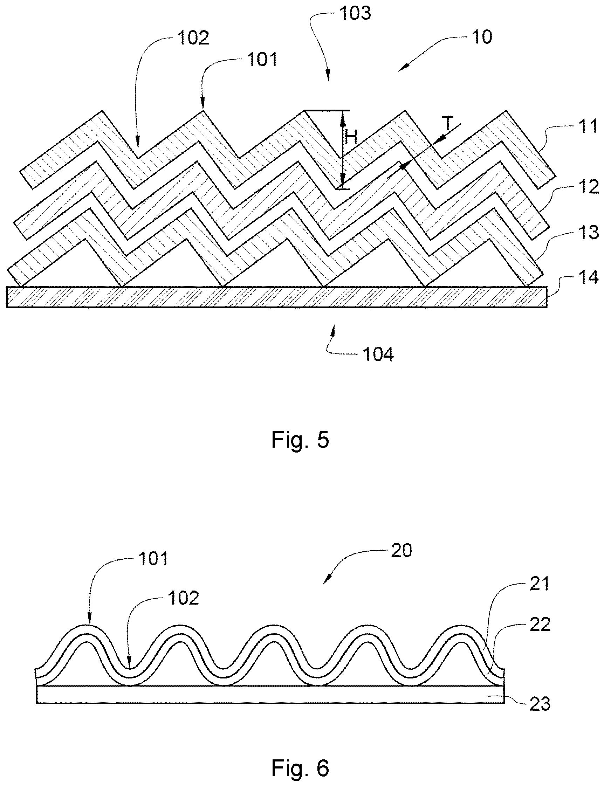

According to one aspect of the present invention, the filter media is a multilayer filter media. Referring to Fig. 5, the multilayer filter media 10 comprises a plurality of filter layers 11-14 which are preferably attached to one another. One or more layers, such as layers 11-13 can have a wavy shape, referred to as a waved layer. A waved layer features alternating peaks 101 and valleys 102. The peaks 101 project from the valleys over a height H which is typically several (at least two) times the thickness T of the substrate of the waved layer. A waved layer may have a uniform layer thickness. Additionally, one or more layers, such as layer 14, can be a flat or planar layer, i.e. a layer that does not comprise any wavy surface. Layer 14 is advantageously used as a backing or support layer, e.g. providing support for the waved layers 11-13. The waved shape of layers 11-13 can be obtained by undulating the substrate or by a plurality of pleats. Such a multilayer filter media can have a first face 103 defining a first exposed surface and a second face 104 defining a second exposed surface opposite the first face 103. The filter media 10 may have a predetermined orientation to air flow, e.g. the filter media can be placed in an air stream such that the first face 103 is arranged at the upstream side (i.e. exposed to the air flow that is to be filtered - dust side), while the second face 104 is arranged at the downstream side (i.e. the clean or filtered side).

Referring to Fig. 6, another example of a multilayer filter media 20 comprises one or more waved layers 21, 22 having an undulated shape, resembling a sinusoidal cross sectional shape. Layers 21, 22 can be attached to each other and to a backing layer 23, which may be flat. The layers may be attached by needle stitching and/or by lamination.

The various layers 11-14 and 21-23 within filter media 10, 20 advantageously have varying density and/or porosity. This may enhance the air filtration characteristic of the filter media. Advantageously, the outer layers 11, 14 of filter media 10 and the outer layers 21, 23 of filter media 20 have a lower density than an inner layer 12 or 22 respectively. By way of example, the ratio of density of inner layer 12 or 22 to the density of outer layer 11, 21 (or 14, 13 or 23) is at least 1.2, preferably at least 1.4.

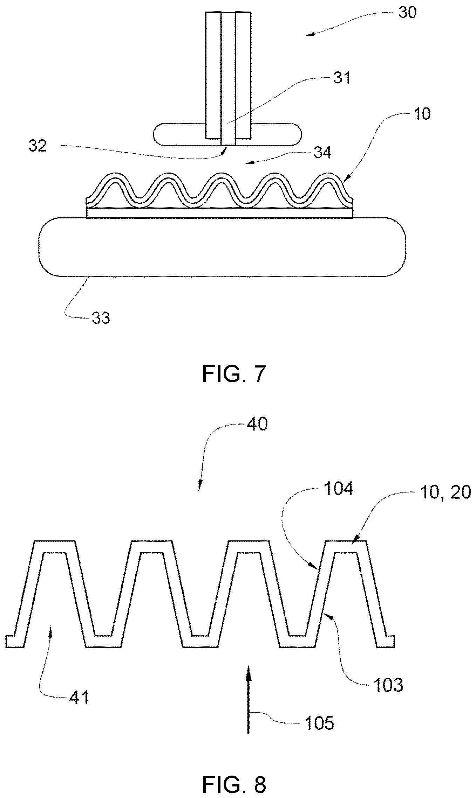

As an advantage, in methods of the present invention, the multilayer filter media 10, 20 is exposed to the plasma discharge or to the plasma afterglow resulting therefrom. Referring to Fig. 7, the filter media 10, 20 can be arranged on a support platform 33, which can e.g. be a conveyer belt. Support platform 33 is arranged opposite the outlet 32 of a plasma chamber 31 of an atmospheric pressure plasma apparatus 30, such as a plasma jet or plasma torch. Filter media 10, 20 can be arranged in the plasma afterglow zone 34 extending between the outlet 32 and the support platform 33. Possibly, the filter media is placed with the backing layer 14, 23 facing the support platform 33, while the outer waved layer 11, 21 is facing the outlet 32. Alternatively, the filter media can be placed on the support platform 33 upside down, i.e. with backing layer 14, 23 facing the outlet 32. Advantageously, the filter media is processed twice through the plasma apparatus 30, e.g. a first time with outer layer 11, 21 facing outlet 32, and a second time with the filter media inversed (backing layer 14, 23 facing outlet 32).

By so doing, a plurality of layers of the multilayer filter media can be plasma treated at once. As will be shown below, it has been observed that the coating can be deposited into the inner layers of the multilayer filter media, providing a more efficient filter media for inhibiting biological pathogens while the air permeability and air filtration characteristics of the filter media are not affected.

Such multilayer filter media are advantageously used in HVAC systems. In one advantageous application, referring to Fig. 8, the filter media 10, 20 is used in a pocket filter 40, where the filter media is disposed to form a plurality of pockets 41 which can have trapezoidal cross sectional shape. The size of these pockets are advantageously several orders of magnitude larger than the size of the waves of the multilayer filter media. The filter media, e.g. filter media 10, is arranged in the filter 40 such that the first (upstream) face 103 of the filter media is exposed to the dust-laden air flow 105, and the second (downstream) face 104 is exposed to the clean side of the filter.

### Tests and examples

### Example 1

The coatings in the tests described below were obtained using a PlasmaSpot^{®} apparatus by Molecular Plasma Technology, Luxembourg, using a power around 100 W (but any power between 60 - 450 W can be used), a voltage about 15 kV (but any voltage between 5 and 25 kV can be used), a frequency about 60 kHz, plasma gas N₂, (but also N₂, He, Ar, even air and mixtures thereof can be used, although inert gasses are preferred), a plasma gas flow of about 100 slm (but any plasma flow between 10 and 250 slm can be used), the precursor injected as aerosol (but a liquid precursor, gas precursor, a mixture of liquids, solutions, dispersions, etc. also work well), a working distance about 8mm (but any working distance between 1 and 20 mm seems to work), a temperature about room temperature.

### 1. Virucidal tests with MS2

The biological pathogen transfer inhibiting properties of the coating were tested for a multitude of precursors. The testing below was performed using Bacteriophage MS2. Bacteriophage MS2 is a 275 Å RNA virus that infects Escherichia coli. Because of its small size, relatively simple composition and ease of growth, MS2 is used as a model organism for a number of macromolecular processes including viral replication, translation, infection, and assembly. Increasingly due to its ease of purification, harmlessness to mankind, and durability, MS2 is also used as a quantitative marker for the effectiveness of antiviral and antiseptic agents, and the efficiency of water treatment plants and filtration devices. Additionally, genetically modified forms of MS2 are available for vaccine development and for use as clinical diagnostic tools. Testing on MS2 is commonly used to look at the effects a procedure has on enveloped viruses in general. In the present case, the test involves subjecting a substrate having a bio-active layer deposited using a method according to the present invention, to MS2 contamination.

The tests were performed at the LIST (Luxembourgish Institute for Science and Technology).

A viral load reduction test was performed according to the ASTM E2721-16 standard. The Influenza A virus which is proposed in ASTM E2721-16 was replaced as model by another virus: MS2. MS2 is a bacteriophage preventing any biosafety risk during testing. Results on the virucidal activity are obtained faster (24 h) than with Influenza A (3-4 days). MS2 are naked viruses used in other standards regarding virucidal effects (e.g. EN 14476 - Quantitative suspension test for the evaluation of virucidal activity in the medical area).

The test involved a validation part and a data gathering part. In the control part, MS2 phages were sprayed on a surface of an untreated filter media, i.c. the surface of a PPE mouth mask. Recovery of MS2 phages is obtained by rinsing the filter media, i.c. the PPE mouth mask. The data gathering part involves a treatment step prior to the spraying and rinsing step also used in the control part. The treatment step hereby entails performing the method of the present invention for a number of embodiments. The effects can be obtained by comparing the results of the data gathering part with those of the validation part.

In terms of infection control, 'Log Reductions' convey how effective a product is at reducing pathogens. The greater the log reduction the more effective the product is at killing bacteria and other pathogens that can cause infections.

During product efficacy testing, microbiology laboratories count the number of colony forming units (CFUs) in the case of bacterial testing or plaque forming units (PFUs) in case of viruses present at the start of the test. The tests are performed on a treated substrate being tested, alongside a control substrate and wait the required test time before counting the number of CFUs or PFUs present.

The result of the difference between the control and the test substrate is then expressed as a Log reduction. For example, if the number of CFUs in the control was found to be 1,000,000 (or 10⁶) and the end result using the product was only 1,000 (10³), that would be a Log reduction of 3 or a reduction of 99.9%.

The results of the tested precursors on Tyvek^{®} substrates are summarized in Table 3. Tyvek^{®} is a 100% synthetic material made from high-density spunbound polyethylene fibers. Tyvek^{®} is commonly used in many applications, in particular in personal protective equipment.

Herein, "LOD" refers to "Limit Of Detection" of the testing method. "EPA Safer Chemical List" refers to whether the compound can be found in the Safer Chemical Ingredients List of the Environmental Protection Agency. "EPA Covid" refers to a list of compounds which are deemed effective against the Sars-Cov-2 virus and the Covid-19 disease.

**Table 3**

| Precursor or precursor mixture | CAS No. | EPA Covid | EPA Safer Chemical List | **LOG Reduction** |
|---|---|---|---|---|
| Citric Acid (pH mod.) | 77-92-9 | Yes | Yes | **> 5.0 (LOD)** |
| Benzal | 63449-41-2 | Maybe | No | **> 3.8 (LOD)** |
| Antibak Residual | 68439-45-2 | No | No | **> 2.9 (LOD)** |
| | 141-43-5 | | | |
| | 7173-51-5 | | | |
| | 68424-85-1 | | | |
| APTAC | 45021-77-0 | Maybe | No | **2.1** |
| SiQAM | 27668-52-6 | Maybe | No | **1.56** |
| Chitosan | 9012-76-4 | No | Yes | **2.6** |
| Gold nanoparticle AuNP (additive) | 7440-57-5 | No | No | **> 2.9 (LOD)** |
| CuO (additive) | 1317-38-0 | No | No | **> 3.8 (LOD)** |

Antibak Residual comprises ethoxylated C6-C12 alcohols, 2-aminoethanol, Didecyldimethylammonium chloride and N-benzyl-N,N-dimethyltetradecan-1-aminium chloride (CAS numbers provided in the table). ATAC refers to 2-(Dimethylamino)ethyl acrylate, methyl chloride quaternary salt. APTAC refers to (3-Acrylamidopropyl)trimethylammonium chloride which is preferably in solution.

The results in the above table indicate that the tested precursors lead to a LOG reduction, indicating their effectiveness in inhibiting the transfer of the biological pathogen. Note that these tested precursors belong to a multitude of classes. Citric acid is an organic acid; benzal, SiQAM and APTAC are ammonium chlorides, Antibak residual is a mixture of alcohols and ammonium chlorides, ATAC is a quaternary salt, chitosan is a natural antibacterial substance, AuO and CuO are metal nanoparticles. Their effectiveness for inhibiting transfer of a biological pathogen when coated using a method of the present invention, and in particular for their antiviral or virucidal properties, is illustrative for other precursors of the same class.

### 2. Anti-bacterial tests with Staphylococcus aureus and Escherichia coli

Tests were performed on a polypropylene nonwoven (PPNW) substrate having a density of 25 g/m², which is commonly used as the outer layer of a surgical face mask, and on the synthetic textile Tyvek^{®}.

Droplets containing bacterial suspension are placed on the treated material and evaluated after an incubation period. After the incubation, the bacteria are recovered from the tested material by washing it with a recovery medium. This recovery medium is then analysed for presence of the pathogen by incubation of the recovery medium on agar plates.

If the material has antibacterial properties, the amount of bacterial colony forming units on the agar plate will be greatly reduced when comparing with the untreated reference material.

The bacteriae used in the testing were Staphylococcus aureus (gram-positive) and Escherichia coli (gram-negative).

The test is based on the international standard given by OECD guidelines for testing of chemicals based on ISO standards 22197/20743: Quantitative method for evaluating antibacterial activity of porous and non-porous antibacterial treated materials.

Samples size is 100 mm², Bacterial strain is S.aureus ATCC6538P, volume of inoculum is 0.2 ml (40 X 5 µl), number of viable bacteria in the test inoculum is 6.60E+05 CFU/ml (colony forming units/ml), neutraliser is SCDLP (ISO 22196).

Conditions for a valid test: When the three conditions given in (a) and (b) respectively, are satisfied, the test is deemed valid. If all conditions are not met, the test is not considered valid and the samples shall be re-tested.
a) The average number of colony forming units recovered immediately after inoculation from the untreated test samples, is within the range 6.2 × 103 CFU/cm2 to 2.5 × 104 CFU/cm2 for non-porous materials and between 1.2 × 105 CFU/g to 4.5 × 105 CFU/g for porous materials.
   Range for 0.4 g of porous materials: 4.80E+04 to 1.80E+05 CFU total
b) The number of colony forming units recovered from each untreated test sample after incubation for 24 hours will not be less than 6.2 × 101 CFU/cm2 for non-porous materials and 1.2 × 103 CFU/g for porous materials.

Range for 0.4 g of porous material: not less than 480 CFU total

The test results for different precursors on the PPNW substrate are summarized in Table 4 and on the Tyvek^{®} substrate in Table 5.

**Table 4**

| Compound class | Compound | CAS | Gram+ log reduction | % Gram+ bacteria reduced | Gram-log reduction | % Gram-bacteria reduced |
|---|---|---|---|---|---|---|
| Organic acid | Citric acid | 77-92-9 | > 4.3 | > 99.995 | > 5.0 | > 99.999 |
| Ammonium chloride | Benzalkonium chloride | 63449-41-2 | > 4.2 | > 99.994 | 5.4 | 99.9996 |
| | SiQAM | 27668-52-6 | > 4.2 | > 99.994 | > 5.4 | > 99.9996 |
| Alkyl amine | DIMAEMA | 2867-47-2 | > 4.2 | > 99.994 | > 5.4 | > 99.9996 |
| Natural antibacterial substance | Chitosan | 9012-76-4 | > 4.2 | > 99.994 | > 5.4 | > 99.9996 |
| Ammonium chloride with nanoparticles | SiQAM + 1% CuO | 27668-52-6 | 4.2 | 99.994 | 5.4 | 99.9996 |
| | | 1317-38-0 | | | | |
| Commercial disinfectant - iodine based | PVP-I2 | 25665-41-8 | > 4.2 | > 99.994 | > 5.4 | > 99.9996 |

**Table 5**

| Compound class | Compound | CAS | Gram+ log reduction | %Gram+ bacteria reduced | Gram- log reduction | % Gram-bacteria reduced |
|---|---|---|---|---|---|---|
| Organic acid | Citric acid | 77-92-9 | > 3.8 | > 99.98 | 1.6 | 97.5 |
| Ammonium chloride | Benzalkonium chloride | 63449-41-2 | > 3.3 | > 99.5 | 1.9 | 98.7 |
| | SiQAM | 27668-52-6 | > 3.3 | > 99.95 | > 1.9 | > 98.7 |
| Alkyl amine | DIMAEMA | 2867-47-2 | > 3.3 | > 99.95 | 0.1 | 20.6 |
| Ammonium chloride with nanoparticles | SiQAM + 1% CuO | 27668-52-6 | > 3.3 | 99.95 | > 1.9 | 98.7 |
| | | 1317-38-0 | | | | |
| | SiQAM | 27668-52-6 | > 3.3 | > 99.95 | > 1.9 | > 98.7 |

### 3. Phi-6 tests

For this study, the PlasmaSPOT equipment developed by Molecular Plasma Group S.A. was used for its fast setup and reliability to establish proof-of-concept. Citric acid in a 50 wt% aqueous solution was used as precursor. To evaluate the performance of the citric acid plasma-treatment, Phi6 virucidal tests (using an enveloped bacteriophage, a direct surrogate for SARS-Cov-2) were performed at the Luxembourg Institute of Science and Technology (LIST).

Phi-6 bacteriophage is an enveloped virus that is used as a meaningful surrogate for enveloped viruses such as Influenza A or Sars-Cov-2. Phi-6 is a virus belonging to the Cystoviridae family. It typically attaches to the Type IV pilus or Pseudomonas syringae and this latter is therefore used for the detection of infective virions. The modified ISO standard 10705-1:1995 has been used for the present analysis.

The principle is the following: the sample, i.e. a coated filter media material, is mixed with a small volume of semisolid nutrient medium. A culture of host strain is added and plated on a solid nutrient medium. After this, incubation and reading of plates for visible plaques takes place. For the assays, droplets of Phi-6 phage suspension are added, dried onto the surface of test and control samples and held at room temperature for varying contact time (defined for each experiment separately). Viral particles are collected, then assayed for viable virus as described above.

The filter media used was PP nonwoven and tests were performed for two base weights of the filter media (20 g/m² and 38 g/m²). The precursor used was a 50% citric acid aqueous solution provided by Citrique Beige, referred to as "beCA 50% in Table 6..

The results are summarized in table 6, wherein "Passes" refers to the times the filter material is passed underneath the plasma source (or through the plasma afterglow zone).

**Table 6**

| Base weight [g/m²] | Incubation time [min] | Precursor | Equipment | Precursor flow [slm] | Passes | Phi-6 log reduction |
|---|---|---|---|---|---|---|
| 20 | 10 | beCA 50% | PlasmaSpot | 2inj*1.2 | 3 | >4.2 |
| 20 | 30 | | | | 3 | >4.2 |
| 20 | 120 | | | | 3 | >3.8 |
| 20 | 120 | | | | 1 | >3.8 |
| 38 | 120 | | | | 3 | >3.8 |

It was noticed that both the 20 g/m² and the 38 g/m² filter media show good processability with the plasma process and it is clear from Table 6 that high log reductions for the Phi-6 virus surrogate were obtained. These filter media base weights correspond to filter media such as used in the DeltriSafe+ facial mask (20 g/m²) and the DeltriSafe IIR+ facial mask (38 g/m²), both of Deltrian International S.A., Belgium. The log reduction values exceeded the limit of detection of the experiment. Phi-6 Sars-Cov-2 surrogate virus has demonstrated high inactivation levels already after 10 minutes of incubation, exceeding the log reduction limit of detection of the experiment. The virucidal coating retains its activity for the different base weights of the materials tested. Note that the filter material was spunbonded propylene.

### 4. Performance testing (EN 14683)

The facial mask DeltriSafe IIR+ using a coated filter was also tested and found to meet the EN 14683 performance requirements, which include a test on the air permeability (delta P test) which was found to be essentially unaffected by the coating. The conditions of the EN 14683 tests are summarized below:
Standard test used: EN 14683 - annex B (2019) + AC (2019)
Product standard: EN 14683 (2019) + AC (2019)
Number of tested masks: 5
Dimensions of the test specimen: 16.5 cm x 15 cm
BFE area tested: ca. 49 cm² (BFE = bacterial filtration efficiency)
Masks conditioning: 21±5°C and 85±5 RH (relative humidity)
Side of the mask in contact with the bacterial challenge: inner side
Challenge bacterial strain used: Staphylococcus Aureus ATCC6538
Bacterial challenge per test: 1700-3000 CFU
Total test time: 1 min. delivering challenge + 1 min. without challenge (air flow continuing)
Flow rate: 28.3 l/min
Positive control: tests performed with no filter material in the air stream
Negative control: tests performed without challenge
The Bacterial filtration efficiency B is defined as B=(C-T)/T, expressed as a percentage, with C the mean of the total plate counts for the positive control runs and T the total count for the tested mask. B was larger than 99.9% for all tested masks.

The breathability was tested using EN 14683 - annex C (2019) + AC(2019) standard for 5 masks, and at 5 different positions on each of the masks. At each position and for each mask, the delta P was less than 40 Pa/cm², in accordance with the EN 14683 standard. The results are summarized in Table 7 below.

**Table 7**

| | Mask 1 | Mask 2 | Mask 3 | Mask 4 | Mask 5 |
|---|---|---|---|---|---|
| Area A | 32.6 | 32.2 | 34.8 | 30.8 | 31.4 |
| Area B | 33.6 | 34.8 | 29.3 | 36.3 | 33.8 |
| Area C | 34.2 | 34.4 | 32.8 | 32.0 | 29.1 |
| Area D | 34.8 | 36.7 | 30.4 | 32.2 | 39.9 |
| Area E | 34.2 | 33.8 | 35.0 | 37.1 | 34.6 |
| **Average ΔP (Pa/cm²)** | **33.9** | **34.4** | **32.5** | **33.7** | **33.8** |

These delta P values are essentially the same as for untreated masks. The tests further showed a microbial cleanliness of less than 30 cfu/g and non-cytotoxicity of the samples.

### Example 2

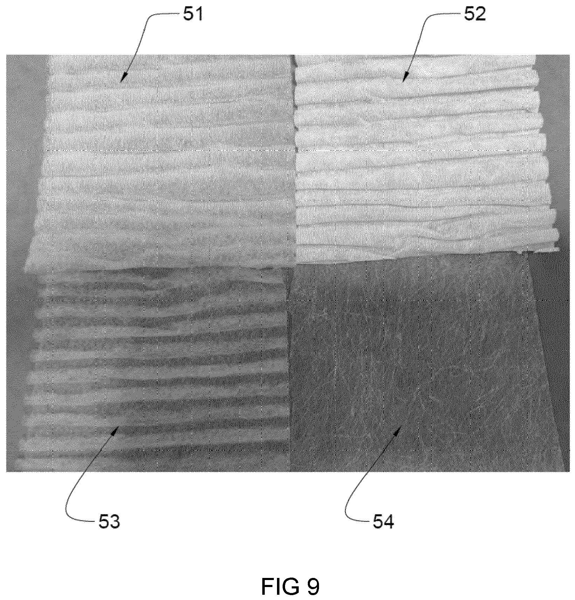

A four-layer filter media type WA0190XT - NanoWave^{®} XT, Hollingsworth & Vose was used. This filter media was composed of a superposition of four layers 51-54 shown separately in Fig. 9, particularly three waved layers 51, 52 and 53 and a backing layer 54 similar to the configuration of Fig. 5. The intermediate layer 52 is the most dense layer, followed by top (outer wave) layer 51 and bottom waved layer 53 (less dense than layer 51). All layers were made of PP fibers. The base weight of the overall filter media was about 180 g/m² (according to ISO 536) and the total thickness about 5 mm.

The filter media was placed with the outer backing layer 54 on the support platform 33 (Fig. 7) during the atmospheric pressure plasma coating process, so that the outer waved layer 51 was exposed to the coating precursor-comprising plasma discharge.

The equipment used was the PlasmaSPOT^{®} equipment, and the coating precursor used was an aqueous solution comprising 20% of citric acid by weight. Coating was applied with varying number of passes of the media through the plasma afterglow zone, respectively 6, 10 and 20 passes.

### 1. Penetration depth

The penetration depth was measured by measuring the change in pH value before and after coating on samples of the outer wave layer 51. The results are presented in Table 8. All the samples showed an overall pH change of at least 1.3, indicating an effective treatment level. Presence of citric acid was also detected in the intermediate layer 52.

### 2. Virucidal testing with Phi-6

Phi-6 bacteriophage is an enveloped virus that is used as a meaningful surrogate for enveloped viruses such as Influenza A or SARS-CoV-2. Phi-6 is a virus belonging to the Cystoviridae family. It typically attaches to the Type IV pilus of Pseudomonas syringae and this latter is therefore used for the detection of infective virions. The modified ISO standard 10705-1:1995 has been used for the present analyses.

The Phi-6 virus test was performed on samples of the outer wave layer to obtain a meaningful virus recovery from the substrate. The samples were mixed with a small volume of semisolid nutrient medium. A culture of host strain was added and was plated on a solid nutrient medium. After this, incubation and reading of plates for visible plaques was performed. For the assays, droplets of Phi-6 phage suspension were added and dried onto the surface of test and control samples and the samples were held at room temperature for a 2-hour contact time. Viral particles were collected and assayed for "viable" virus. All samples showed a neutralisation of the Phi-6 virus of more than 99.37%, thereby passing the test (requirement of 99%), resulting in a Phi-6 log red value of more than 2.2 (well above the minimal value of 2 to pass the test).

### 3. Virucidal testing with MS2

An MS2 test was performed as explained for example 1, on both outer wave layer samples and the entire filter media. It is clear from Table 8 that all samples pass the test (at least 99% MS2 neutralized and log red value of at least 2), except for the 10-pass outer wave sample, which shows a borderline fail. This indicated the excellent virucidal functionality of the plasma coatings obtained from a 20% by weight citric acid aqueous solution as coating precursor.

### 4. Buffer test

A buffer test was performed on both the outer wave samples and the filter media to assess the activity of citric acid on virus medium and to assess the ability of the treated sample to change the pH of the buffered medium. All cell culture media used for virus efficiency tests contain an indicator dye, phenol red. This dye changes colour from red to yellow upon acidification of the liquid.

A way to test the activity of the coating and the acidity effect is to test the colour change of the buffered cell media in a droplet on the coating. When a medium on a treated surface changes the pH and thus the colour of the indicator dye goes to yellow, it means that the level of treatment is high enough to guarantee activity. As shown in Table 8, all tested samples passed the buffer test.

**Table 8**

| **Passes No.** | **Sample** | **Avg. MS2 (log red)** | **Avg. Phi-6 (log red)** | **MS2 neutralized (%)** | **Phi-6 neutralized (%)** | **Buffer Test** | **pH test** |
|---|---|---|---|---|---|---|---|
| 6 | Filter media | 2.15 | - | 99.29 | - | pass | - |
| 10 | Filter media | 2.72 | - | 99.81 | - | pass | - |
| 20 | Filter media | 2.46 | - | 99.65 | - | pass | - |
| 6 | Outer wave | 2.13 | >2.2 | 99.26 | >99.37 | pass | 1.66 |
| 10 | Outer wave | 1.96 | >2.2 | 98.90 | >99.37 | pass | 1.72 |
| 20 | Outer wave | 2.71 | >2.2 | 99.81 | >99.37 | pass | 1.78 |

### Example 3

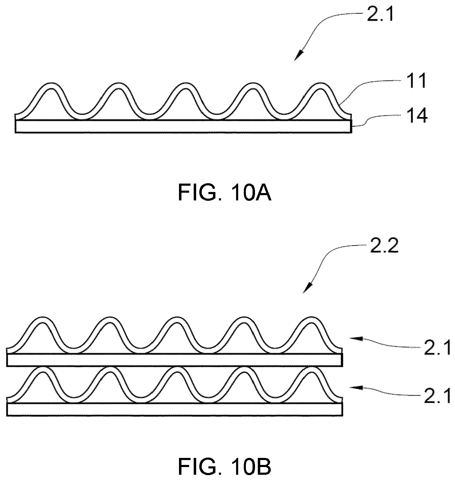

A first two-layer filter media (referred to as media 2.1) was formed by superposition of a waved layer 11 and a planar backing layer 14 as shown in Fig. 10A. Both waved layer and planar backing were made of PS nonwoven. A second four-layer filter media (referred to as media 2.2) was obtained by superposition of two of the first filter media 2.1 as shown in Fig. 10B.

An aqueous solution comprising 40% by weight (wt%) citric acid and 5 mg/liter of a UV marker (Radglo CFF-X-03 (CAS 91-44-1)) was used as coating precursor. An aerosol of the precursor was generated by injecting the precursor solution into 50 slm N₂ (4 injections of 1.2 slm precursor solution). Nitrogen was used as the plasma gas at a flow rate of 500 slm. The total power applied was 1500 W, and the distance between the plasma source and the exposed surface of the substrate was 6 mm. The line speed was 2.4 m/min, and the substrate was passed 10 times through the plasma afterglow area. A Plasmaline^{®} plasma apparatus, Molecular Plasma Group SA, Luxembourg, was used.

### 1. Penetration depth

Using the above parameters, a sample of filter media 2.1 was plasma coated with the wave layer facing the plasma chamber outlet, a second sample of filter media 2.1 was plasma coated with the backing layer facing the plasma chamber outlet, and a third sample consisting of the backing layer alone was plasma coated.

The UV marker was used to visualize the penetration depth of the deposited coating by means of placing the sample under a 365 nm UV source. It was noticed that the coating penetrated the filter media sufficiently, thereby depositing the coating not only on the exposed surfaces but also on the inner surfaces of the filter media.

### 2. Samples prepared for functional testing

Filter media 2.2 were coated according to the process parameters of Table 9 using the same PlasmaLine^{®} equipment. For each coating process, 2 samples were coated with the outer waved layer exposed to the plasma source, and 2 samples were coated with the outer backing layer surface exposed.

Two precursors were used: an aqueous solution comprising 40% by weight of citric acid, and a solution of 15% by weight of chlorocresol in ethanol.

**Table 9**

| **Precursor** | **Power [W]** | **N₂ flow [slm]** | **Precursor flow [slm]** | **Precursor solution flow [slm]** | **Gap [mm]** | **Line speed [m/min]** | **Number of passes** |
|---|---|---|---|---|---|---|---|
| Citric Acid | 1500 | 500 | 8inj*1.2 | 75 | 6 | 2,4 | 5 |
| Citric Acid | 1500 | 500 | 8inj*1.2 | 75 | 6 | 2,4 | 10 |
| Citric Acid | 1500 | 500 | 8inj*1.2 | 75 | 6 | 2,4 | 20 |
| Chlorocresol | 1500 | 500 | 8inj*1 | 75 | 6 | 2,4 | 5 |
| Chlorocresol | 1500 | 500 | 8inj*1 | 75 | 6 | 2,4 | 10 |
| Chlorocresol | 1500 | 500 | 8inj*1 | 75 | 6 | 2,4 | 20 |

### 3. Virucidal testing with MS2

The anti-viral properties were tested by application of a fixed number of water droplets containing the MS2 virus on the surface of the samples. The droplets were applied to the surface that was exposed to the plasma source during the atmospheric pressure plasma coating process. After an incubation of 2 hours, the samples were immerged in a recuperation solution to extract the virus from the samples. The recuperation solution was placed in a petri dish wherein the *E.coli* bacteria grew, and the number of viral zones (zones comprising death bacteria) are counted. A lower number of viral zones indicates a larger neutralisation of the virus by the plasma coating.

Table 10 shows the results. The Virucide result (log value) gives the log value of the number of living viruses found in the recuperation solution.

**Table 10**

| **Precursor** | **Exposed side** | **Passes** | **Virus neutralized (%)** | **Viruside results (log red)** |
|---|---|---|---|---|
| Citric Acid | waved layer | 5 | 80.0 | 0.7 |
| Citric Acid | waved layer | 10 | 93.7 | 1.2 |
| Citric Acid | waved layer | 20 | 98.7 | 1.9 |
| Chlorocresol | waved layer | 5 | 36.9 | 0.2 |
| Chlorocresol | waved layer | 10 | 74.9 | 0.6 |
| Chlorocresol | waved layer | 20 | 84.2 | 0.8 |
| Citric Acid | Backing layer | 5 | 93.7 | 1.2 |
| Citric Acid | Backing layer | 10 | 96.0 | 1.4 |
| Citric Acid | Backing layer | 20 | 99.8 | 2.7 |
| Chlorocresol | Backing layer | 5 | 68.4 | 0.5 |
| Chlorocresol | Backing layer | 10 | 98.0 | 1.7 |

It follows from Table 10 that the filter media samples having their outer backing layer exposed to the plasma source show better results than for the outer waved layer exposed. Further, as is visible in particular for the samples with the exposed waved layer, better results are obtained for filter media coated by passing 10 and 20 times through the plasma afterglow zone than for passing 5 times.

It is also clear that better results are obtained with the citric acid coating than with the chlorocresol coating, in particular for the filter media with the nanowave side exposed to the plasma source.

### Example 4

Same filter media 2.2 as in example 3 was used. The filter media was placed with the outer backing layer on a support platform and the outer wave layer exposed to the coating precursor-comprising plasma discharge (or the plasma source) during the atmospheric pressure plasma coating process.

Two different precursors were used: an aqueous solution comprising 50% by weight of citric acid (CAS 77-92-9), and an aqueous solution comprising 25% by weight n-alkyl(C12-16)-n,n-dimethyl-n-benzylammonium chloride (BTC50E; CAS 68424-85-1) (1:1 w/w dilution in water).

Filter media samples were coated with each coating by passing 3 or 6 times through the plasma discharge area. The virucidal performance was tested by the MS2 test as explained above. An accelerated ageing test was performed according to ASTM F1980-16. The samples were heated to 55°C and 75% relative humidity for 3 days and 6 days, after which a MS2 test was performed. An ageing test of 3 days at 55°C and 75% RH represents 1 month in real life conditions. The results of the virucidal performance after ageing gives an idea of the durability of the coating over time. The results are presented in Table 11.

**Table 11: ageing tests for virucidal performance of coated filter media (N/A: not available)**

| **Time real life (months)** | **Time oven (days)** | | **Citric acid 3 passes** | **Citric acid 6 passes** | **BTC50E 3 passes** | **BTC50E 6 passes** |
|---|---|---|---|---|---|---|
| 0 | 0 | **Virus neutralized (%)** | 99.86 | 99.78 | 99.57 | 99.83 |
| | | **Viruside results (log red)** | 2.86 | 2.65 | 2.37 | 2.78 |
| 1 | 3 | **Virus neutralized (%)** | N/A | N/A | 99.96 | 99.82 |
| | | **Viruside results (log red)** | N/A | N/A | 3.38 | 2.75 |
| 2 | 6 | **Virus neutralized (%)** | N/A | 98.09 | 99.74 | 99.83 |
| | | **Viruside results (log red)** | N/A | 1.72 | 2.59 | 2.78 |
| 3 | 9 | **Virus neutralized (%)** | 97.76 | 96.45 | 99.35 | 98.88 |
| 6 | 19 | **Virus neutralized (%)** | 97.38 | 95.91 | 99.86 | 99.83 |
| 9 | 27 | **Virus neutralized (%)** | 99.00 | 97.61 | 99.89 | 99.70 |
| 12 | 36 | **Virus neutralized (%)** | 97.91 | 93.97 | 99.98 | 98.59 |

From Table 11 it follows that the virucidal properties of the both coatings does not seem to be affected by humidity for up to 36 days testing (12 months in real life conditions), as the variations in the results are in the range of the error margin of the MS2 testing.

Further, it seems that a thicker coating, represented by 6 passes, does not lead to significantly better results than the thinner coating, represented by 3 passes. Hence, it seems that a coating with limited thickness would provide sufficient virucidal properties to the filter media, even over time. Further, as explained above, a thinner coating is in general advantageous to limit the reduction in air permeability and the increase in pressure drop of the filter media.

From table 11 it further appears that the alkyldimethylbenzylammonium chloride (i.e. benzalkonium chloride) coating shows an improved resistance to humidity degrading over citric acid coatings.

### Example 5: Differential Pressure

The same filter media as in Example 2 was used (Fig. 9) to investigate influence of the plasma coating on differential pressure across the filter media. Both an untreated and plasma coated sample of the filter media were subjected to differential pressure test. The plasma coated sample was obtained by coating with a PlasmaLine^{®} apparatus at 1500 Wand 100 slm flow of N₂. A precursor aerosol was generated by injecting 50 wt% aqueous solution of citric acid into a flow of 50 slm N₂ and an additional vector gas for the aerosol of 50 slm N₂ was used.

For the differential pressure test, a 4-pocket filter was prepared with the uncoated and coated filter media respectively. The 4-pocket filter had a face area of 0.082 m² (square section of 0.287m side length) and a total effective filter area of 0.800 m². Differential pressure (dP) was measured at different flow rates between about 200 m³/h and 1000 m³/h at 40.5% RH and 981.1 hPa atmospheric pressure. Results are summarized in table 12, and from the measurements no difference in differential pressure can be observed between the plasma coated and untreated samples.

**Table 12: differential pressure test results**

| Flow Rate (m³/h) | dP (Pa) untreated sample | dP (Pa) plasma coated sample |
|---|---|---|
| 996.76 | 162 | 160 |
| 789.49 | 121 | 120 |
| 593.59 | 86 | 85 |
| 387.87 | 57 | 57 |
| 196.83 | 32 | 32 |

### Example 6: Fractional Efficiency

The same filter media as in Example 5 was used to investigate the influence of the plasma coating on filter media particle retention efficiency. Both an untreated and a plasma coated sample of the filter media were subjected to a fractional efficiency test according to ISO 16890. Identical 4-pocket filters as in Example 5 were constructed with face area of 0.082 m² and filter area of 0.800 m². During the test, temperature was 20.5°C, relative humidity 42,1% RH and atmospheric pressure 981.5 hPa. Flow rate used was 799,60 m³/h and dust concentration (DEHS) was 30,0 mg/m³.

In the test, a flow with standardized particle distribution concentration is made to flow through the filter, and the amount of particles upstream and downstream of the filter is measured for a given number of particle size ranges. The filter media was placed with the outer wave layer facing the upstream side (dust side) while the outer backing layer is facing the clean air side.

In the particle size range between 0.3 µm and 10 µm a mean retention efficiency of 88,8% was measured for the plasma coated filter media, and 88.7% for the untreated filter media. The efficiency for different particle size ranges is summarized in table 13.

**Table 13: Particle retention efficiency of filter media. Particle retention is measured by counting the amount of particles at the upstream side (Xᵤₚ) and the amount of particles at the downstream side (X_{down} i.e. particles which have passed the filter) and efficiency = Xᵤₚ - X_{down} / Xᵤₚ * 100.**

| Particle size range (µm) | Efficiency untreated sample (%) | Efficiency plasma treated sample (%) |
|---|---|---|
| 0.3 - 0.4 | 80.9 | 81.0 |
| 0.4 - 0.6 | 86.6 | 86.6 |
| 0.6 - 0.7 | 89.3 | 89.3 |
| 0.7 - 1.0 | 95.5 | 95.4 |
| 1.0 - 1.3 | 96.4 | 96.4 |
| 1.3 - 1.6 | 97.2 | 97.2 |
| 1.6 - 2.2 | 97.9 | 97.8 |
| 2.2 - 3.0 | 98.9 | 98.8 |
| 3.0 - 4.0 | 99.2 | 99.2 |
| 4.0 - 5.5 | 99.7 | 99.7 |
| 5.5 - 7.0 | 100.0 | 100.0 |
| 7.0 - 10.0 | 100.0 | 100.0 |

## Claims

1. A method for plasma coating a compressible structure , comprising the steps of:
- compressing the compressible structure, thereby at least partially, and preferably essentially completely, removing air from the compressible structure;
- coating the compressed structure according to the following steps:
i) ionizing a plasma gas at a temperature of 150°C or lower, and at about atmospheric pressure, thereby creating a plasma;
ii) introducing a precursor into said plasma, thereby obtaining a precursor-comprising plasma;
iii) exposing the compressed structure to said precursor-comprising plasma, thereby forming a coating onto surfaces of the structure.

2. A method according to claim 1, wherein the compressible structure is mechanically compressed.

3. A method according to claim 1 or 2, wherein the compressed structure decompresses during exposure of the compressed structure to the precursor-comprising plasma.

4. A method according to any of the preceding claims, wherein the precursor comprises a biological pathogen transfer inhibiting compound.

5. A method according to claim 4, wherein the biological pathogen transfer inhibiting compound is a biological pathogen inactivation compound, a biological pathogen immobilisation compound and/or a biological pathogen proliferation decreasing compound.

6. A method according to claim 5, wherein the biological pathogen transfer inhibiting compound is a virucidal compound.

7. A method according to claim 6, wherein the precursor is or comprises citric acid or alkyldimethylbenzylammonium chloride.

8. Method according to any of the preceding claims, whereby said plasma gas comprises inert gas for at least 99 % by volume, preferably whereby said inert gas is N2, and/or whereby said plasma gas comprises O2 for at most 1% by volume.

9. Method according to any of the preceding claims, wherein the precursor is introduced in a plasma gas afterglow of said plasma.

10. Method according to any of the preceding claims, wherein the precursor is administered in the plasma as an aerosol.

11. A method according to any of the preceding claims, wherein the compressible structure is air-permeable, preferably wherein the compressible structure is an air-permeable filter.

12. Method according to claim 11, wherein the air permeability of the coated structure has decreased with respect to the air permeability of the uncoated structure for 20% or less, more preferably 15% or less, still more preferably 10% or less, yet more preferably 8% or less, even more preferably 6% or less, such as 5% or less, 4% or less, 3% or less, 2% or less or, most preferably 1% or less.

13. A method according to any of the preceding claims, the compressed structure is exposed to said precursor-comprising plasma from at least two sides simultaneously.

14. Method according to any of the preceding claims, wherein the structure comprises a thickness of at least 1 mm or wherein the structure comprises a thickness of at least 3 cm.

15. A compressible structure comprising a coating obtained using the method according to any of the previous claims.
